# EUROPÄISCHE PATENTSCHRIFT

(11) **EP 3 818 038 B1**
(45) Veröffentlichungstag und Bekanntmachung des Hinweises auf die Patenterteilung: **03.08.2022**
(21) Anmeldenummer: 19733807.2
(22) Anmeldetag: 01.07.2019
(51) Int. Cl.: C07B 37/04, C07B 43/04, C07C 209/22, C07C 213/08, C07C 303/30, C07D 209/82, C07D 405/04, C07C 211/54, C07C 211/55, C07C 211/56, C07C 211/58, C07C 309/65

(54) **VERFAHREN ZUR HERSTELLUNG VON ARYLAMINEN**
PROCESS FOR THE PRODUCTION OF ARYLAMINES
PROCÉDÉ DE PRÉPARATION D'ARYLAMINES

(30) Priorität: 04.07.2018 EP 18181706
(43) Veröffentlichungstag der Anmeldung: 12.05.2021
(73) Patentinhaber: Merck Patent GmbH, 64293 Darmstadt (DE)
(72) Erfinder: EHRENREICH, Christian, 64285 DARMSTADT (DE); JOOSTEN, Dominik, 64372 OBER-RAMSTADT (DE); FACKLER, Philipp Hans, 60486 Frankfurt am Main (DE); VOM STEIN, Thorsten, 64289 Darmstadt (DE); LEHMANN, Stefan, 64853 Otzberg (DE); WERN, Caroline, 66564 OTTWEILER (DE)
(86) Internationale Anmeldenummer: PCT/EP2019/067535
(87) Internationale Veröffentlichungsnummer: WO 2020/007770

(56) Entgegenhaltungen:
- WO-A1-2017/148565
- MAXIM A. TOPCHIY ET AL: "Solvent-Free Buchwald-Hartwig Reaction of Aryl and Heteroaryl Halides with Secondary Amines : Solvent-Free Buchwald-Hartwig Reaction", EUROPEAN JOURNAL OF ORGANIC CHEMISTRY, Bd. 2014, Nr. 16, 1. Juni 2014 (2014-06-01), Seiten 3319-3322, XP55618578, DE ISSN: 1434-193X, DOI: 10.1002/ejoc.201402077
- CHIL WON LEE ET AL: "Improved power efficiency in blue phosphorescent organic light-emitting diodes using diphenylmethyl linkage based high triplet energy hole transport materials", ORGANIC ELECTRONICS., Bd. 14, Nr. 1, 1. Januar 2013 (2013-01-01) , Seiten 370-377, XP55618537, NL ISSN: 1566-1199, DOI: 10.1016/j.orgel.2012.11.004
- LOUIE J ET AL: "Palladium-Catalyzed Synthesis of Arylamines from Aryl Halides. Mechanistic Studies Lead to Coupling in the Absence of Tin Reagents", TETRAHEDRON LETTERS, ELSEVIER, AMSTERDAM, NL, Bd. 36, Nr. 21, 22. Mai 1995 (1995-05-22), Seiten 3609-3612, XP004028031, ISSN: 0040-4039, DOI: 10.1016/0040-4039(95)00605-C
- YUHKI OHTSUKA ET AL: "Palladium-Catalysed Amination of Hindered Aryl Halides with 9 H -Carbazole", SYNTHETIC COMMUNICATIONS, Bd. 49, Nr. 1, 2. Januar 2019 (2019-01-02) , Seiten 159-165, XP55619024, PHILADELPHIA, PA; US ISSN: 0039-7911, DOI: 10.1080/00397911.2018.1546402

## Beschreibung

Die Erfindung betrifft ein Verfahren zur Herstellung von Verbindungen enthaltend mindestens eine Arylaminogruppe durch palladiumkatalysierte Kupplungsreaktion einer Aminoverbindung mit einer Arylverbindung.

Das Knüpfen einer Bindung zwischen einem Stickstoffatom und einer Aryl- bzw. Heteroarylgruppe stellt eine Schlüsselreaktion in der organischen Synthese dar. Entsprechend sind Aryl- bzw. Heteroarylaminoverbindungen oftmals wichtige Zwischenprodukte in mehrstufigen Synthesen. Weiterhin finden Aryl- bzw. Heteroarylaminoverbindungen Verwendung als pharmazeutische Wirkstoffe, sowie als funktionelle Materialien, beispielsweise in elektronischen Vorrichtungen. In allen Fällen ist das Erzielen einer hohen Ausbeute im Kupplungsschritt, sowie das Vermeiden von Nebenprodukten von großer Bedeutung, da andernfalls Synthesen im industriellen Maßstab nur schwer realisierbar sind. Weiterhin ist es zur Erzielung einer hohen Produktreinheit unabdingbar, dass Nebenreaktionen weitestgehend unterbleiben. Wünschenswert ist weiterhin eine hohe Selektivität bei der Anwesenheit weiterer funktioneller Gruppen.

Im Stand der Technik sind Verfahren zur Synthese von Arylaminoverbindungen bekannt, bei denen als Edukte eine Aminoverbindung und eine Arylverbindung unter Palladiumkatalyse in Anwesenheit einer Base zur Reaktion gebracht werden (Hartwig-Buchwald-Kupplung). Solche Verfahren sind unter anderem beschrieben in WO2017/148565, in Maxim A. Topchiy et al.(European Journal of Organic Chemistry 2014, 16, 3319), in Chil Won Lee et al. (Organic Electronics 14, 1, 370), in US 5,576,460, in Guram et al. (Angew. Chem., Int. Ed. 1995, 43, 1348), in Louie et al. (Tetrahedron Lett. 1995, 36, 3609), sowie in Surry et al. (Chemical Science 2011, 2, 27). Im Stand der Technik bekannte Versuche zur Weiterentwicklung der Methode betreffen überwiegend die Verwendung neuartiger Ligand-Katalysatorsysteme (Surry et al., Chemical Science 2011, 2, 27). Gemäß dem Stand der Technik wird in der Hartwig-Buchwald-Reaktion typischerweise NaOtBu eingesetzt (siehe o. g. Literaturstellen oder Kuwano et al., Synlett 2010, 1819). Weiterhin ist die Verwendung anorganischer Basen wie KOH, NaOH, CS₂CO₃ oder K₃PO₄ bekannt (siehe o. g. Literaturstellen), ebenso wie die Verwendung sehr starker Basen, wie Alkaliamide oder Alkyllithium (WO 2013/068075).

Trotz der insgesamt guten Effizienz dieser Verfahren und ihrer breiten Anwendbarkeit besteht Bedarf an einer Verbesserung der Methode, insbesondere in Hinblick auf langsam reagierende und/oder sterisch anspruchsvolle Edukte, sowie im Hinblick auf hydrolyseempfindliche Edukte, beispielsweise solche mit Triflaten oder anderen Sulfonaten als Abgangsgruppe. Weiterhin besteht Bedarf an einer Verbesserung des Verfahrens in Hinblick auf Produktausbeute sowie Verringerung der Bildung von Nebenprodukten, insbesondere der Bildung der defunktionalisierten Arylverbindung. Die genannten Probleme treten insbesondere beim Einsatz sterisch gehinderter Edukte auf, beispielsweise ortho-substituierter Arylverbindungen oder sekundärer Aminoverbindungen mit sterisch anspruchsvollen Substituenten. Die Bildung der hydrolysierten Arylverbindung als Nebenprodukt tritt insbesondere bei der Verwendung von Triflat oder anderen Sulfonaten als Abgangsgruppe auf. Auch eine Verbesserung der Selektivität bei Anwesenheit mehrerer unterschiedlicher Gruppen, die als Abgangsgruppe dienen können, ist wünschenswert.

Überraschend wurde gefunden, dass sich Lithium-tert-butanolat (LiOtBu) als Base hervorragend zur Verwendung in palladiumkatalysierten C-N-Kupplungsreaktionen (Hartwig-Buchwald-Kupplung) für die Kupplung von Aryl- bzw. Heteroarylaminen eignet. Dies gilt insbesondere bei Verwendung von Sulfonaten als Abgangsgruppe. Im Vergleich zur Verwendung von NaOtBu, welches typischerweise in C-N-Kupplungsreaktionen verwendet wird, oder KOtBu lassen sich dadurch deutlich verbesserte Ausbeuten erzielen. Gleichzeitig werden bei Verwendung von Sulfonaten als Abgangsgruppe deutlich weniger Nebenreaktionen beobachtet, insbesondere deutlich weniger Hydrolyse der Sulfonatabgangsgruppe. Weiterhin ermöglicht die Verwendung von LiOtBu als Base eine selektive Umsetzung von Sulfonatgruppen, insbesondere von Triflat, in Gegenwart von Chlorsubstituenten. Eine derartige Selektivität wird mit NaOtBu als Base nicht beobachtet.

Die Verwendung von LiOtBu als Base für die Kupplung aliphatischer Amine wird von Louie et al. (Tetrahedron Lett. 1995, 36, 3609) bereits beschrieben. Bei dieser Reaktion mit aliphatischen Aminen wird jedoch nahezu kein Umsatz erhalten.

Gegenstand der Erfindung ist daher ein Verfahren zur Herstellung einer sekundären bzw. tertiären Arylaminoverbindung durch eine palladium-katalysierte Kupplungsreaktion zwischen einer primären bzw. sekundären Arylaminoverbindung und einer Arylverbindung, dadurch gekennzeichnet, dass das Verfahren in Anwesenheit von Lithium-tert-butanolat (LiOtBu) als Base durchgeführt wird.

Dabei führt der Einsatz einer primären Arylaminoverbindung, je nach Katalysator, zu einer sekundären oder tertiären Arylaminoverbindung, und der Einsatz einer sekundären Arylaminoverbindung führt zu einer tertiären Arylaminoverbindung als Reaktionsprodukt. In einer bevorzugten Ausführungsform der Erfindung wird bei dem erfindungsgemäßen Verfahren eine sekundäre Arylaminoverbindung eingesetzt, und es handelt sich bei dem Produkt um eine tertiäre Arylaminoverbindung.

Unter einer Arylaminoverbindung im Sinne der vorliegenden Erfindung wird ein Amin verstanden, an das mindestens ein optional substituiertes aromatisches bzw. heteroaromatisches Ringsystem gebunden ist. Dabei ist eine primäre Arylaminoverbindung eine Verbindung, enthaltend eine primäre Aminogruppe -NH₂, welche an ein optional substituiertes aromatisches oder heteroaromatisches Ringsystem gebunden ist. Eine sekundäre Arylaminoverbindung ist eine Verbindung, enthaltend eine sekundäre Aminogruppe -NH-, welche an zwei optional substituierte aromatische oder heteroaromatische Ringsysteme gebunden ist. Dabei kann auch ein aromatisches Ringsystem und ein heteroaromatisches Ringsystem an den Stickstoff gebunden sein. Eine tertiäre Arylaminoverbindung ist eine Verbindung, enthaltend ein tertiäres Stickstoffatom N, welches an drei optional substituierte aromatische oder heteroaromatische Ringsysteme gebunden ist. Dabei kann auch ein aromatisches Ringsystem und zwei heteroaromatische Ringsysteme oder zwei aromatische Ringsysteme und ein heteroaromatisches Ringsystem an den Stickstoff gebunden sein. Dabei können die aromatischen oder heteroaromatischen Ringsysteme gleich oder verschieden sein. Weiterhin können die aromatischen oder heteroaromatischen Ringsysteme in dem sekundären oder tertiären Amin auch über eine Einfachbindung oder eine bivalente Gruppe miteinander zu einem Ring verknüpft sein. So ist beispielsweise auch die Reaktion eines Carbazols möglich.

Unter einer Arylverbindung wird im Rahmen der vorliegenden Erfindung eine optional substituierte organische Verbindung enthaltend mindestens ein aromatisches bzw. heteroaromatisches Ringsystem verstanden. Dabei enthält die Arylverbindung eine Abgangsgruppe, welche in der erfindungsgemäßen Kupplungsreaktion abgespalten wird. Bevorzugte Abgangsgruppen werden hinten aufgeführt.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem, bevorzugt 6 bis 40 C-Atome. Ein heteroaromatisches Ringsystem im Sinne dieser Erfindung enthält 1 bis 60 C-Atome, bevorzugt 1 bis 40 C-Atome, und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (bevorzugt weniger als 10% der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einer cyclischen Alkyl-, Alkoxy- oder Thioalkoxygruppe im Sinne dieser Erfindung wird eine monocyclische, eine bicyclische oder eine polycyclische Gruppe verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Thiazin, Oxazin, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Isothiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, Isoxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin, Cumarin und Benzothiadiazol.

Unter der Formulierung, dass zwei oder mehr Reste bzw. Gruppen miteinander einen Ring bilden können, soll im Rahmen der vorliegenden Beschreibung unter anderem verstanden werden, dass die beiden Reste bzw. Gruppen miteinander durch eine chemische Bindung unter formaler Abspaltung von zwei Wasserstoffatomen verknüpft sind. Dies wird durch das folgende Schema verdeutlicht.

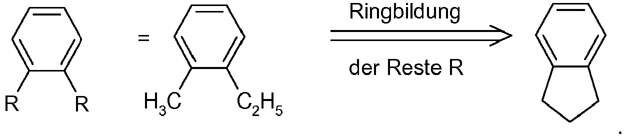

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

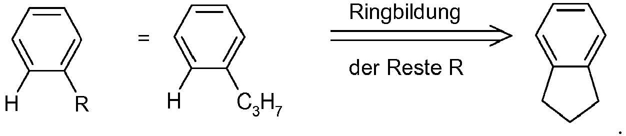

Unter einer palladiumkatalysierten Kupplungsreaktion wird im Rahmen der vorliegenden Erfindung eine Reaktion zwischen zwei organischen Verbindungen verstanden, bei der unter Palladiumkatalyse eine Einfachbindung zwischen den beiden Verbindungen gebildet wird. Dies erfolgt unter formaler Abspaltung eines kleinen Moleküls. Bevorzugt treten dabei keine weiteren Reaktionen an den beiden Verbindungen auf. Die Kupplungsreaktion erfolgt erfindungsgemäß zwischen einer Arylaminoverbindung und einer Arylverbindung, wobei die Arylverbindung eine Abgangsgruppe aufweist, welche bevorzugt ausgewählt ist aus der Gruppe bestehend aus optional substituiertem Alkylsulfonat, optional substituiertem Arylsulfonat, Halogenid und Diazonium. In der Kupplungsreaktion tritt die gebildete Einfachbindung an die Stelle der N-H-Bindung der Arylaminoverbindung und an die Stelle der Bindung zur Abgangsgruppe der Arylverbindung.

Eine bevorzugte Ausführungsform des erfindungsgemäßen Verfahrens entspricht dem folgenden Schema: 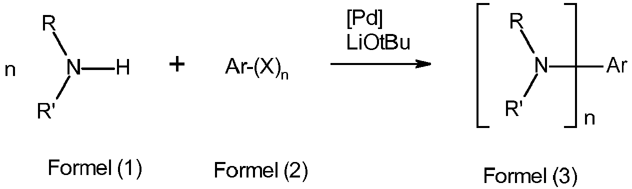 wobei für die verwendeten Symbole und Indizes gilt:
- Ar: ist ein aromatisches oder heteroaromatisches Ringsystem mit 5 bis 60 aromatischen Ringatomen, das mit einem oder mehreren Resten R¹ substituiert sein kann;
- R: ist gleich oder verschieden bei jedem Auftreten ein aromatisches oder heteroaromatisches Ringsystem mit 5 bis 60 aromatischen Ringatomen, das jeweils durch einen oder mehrere Reste R¹ substituiert sein kann;
- R': ist gleich oder verschieden R oder steht für H oder D; dabei können R und R' direkt oder über eine Gruppe R¹ miteinander verknüpft sein und so zusammen mit dem Stickstoffatom, an das sie binden, ein Ringsystem bilden;
- R¹: ist gleich oder verschieden bei jedem Auftreten ausgewählt aus H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 5 bis 30 aromatischen Ringatomen, das jeweils durch einen oder mehrere Reste R² substituiert sein kann, oder einer Aryl- oder Heteroaryloxygruppe mit 5 bis 30 aromatischen Ringatomen, die durch einen oder mehrere Reste R² substituiert sein kann; dabei können zwei oder mehr Reste R¹ miteinander verknüpft sein und so einen Ring bilden;
- R²: ist gleich oder verschieden bei jedem Auftreten ausgewählt aus H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 5 bis 30 aromatischen Ringatomen, das jeweils durch einen oder mehrere Reste R³ substituiert sein kann, oder einer Aryl- oder Heteroaryloxygruppe mit 5 bis 30 aromatischen Ringatomen, die durch einen oder mehrere Reste R³ substituiert sein kann; dabei können zwei oder mehr Reste R² miteinander verknüpft sein und so einen Ring bilden;
- R³: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und so einen Ring bilden;
- X: ist bei jedem Auftreten gleich oder verschieden eine Abgangsgruppe;
- n: ist eine ganze Zahl von 1 bis 10.

Dabei stellt die Verbindung der Formel (1) 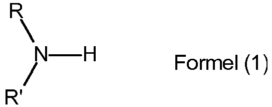 die oben genannte primäre (für R' = H oder D) bzw. sekundäre (für R' gleich oder verschieden R) Arylaminoverbindung dar.

Die Verbindung der Formel (2), Ar-(X)ₙ, stellt die oben genannte Arylverbindung dar, wobei X die Abgangsgruppe darstellt.

Die Verbindung der Formel (3) 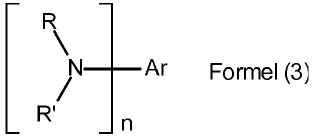 stellt die oben genannte sekundäre (für R' = H oder D) bzw. tertiäre (für R' gleich oder verschieden R) Arylaminoverbindung dar, die das Reaktionsprodukt ist.

Weiterhin steht in der oben aufgeführten Reaktionsgleichung [Pd] für die Palladiumverbindung, die als Katalysator eingesetzt wird.

In einer bevorzugten Ausführungsform ist R' gleich oder verschieden R, so dass bevorzugt eine sekundäre Arylaminoverbindung zur Herstellung einer tertiären Arylaminoverbindung eingesetzt wird.

Das erfindungsgemäße Verfahren eignet sich auch zur Synthese von oligomeren oder polymeren Arylaminoverbindungen. Eine derartige Reaktion kann durch Umsetzung einer Arylverbindung mit mindestens zwei Abgangsgruppen mit einer Arylaminoverbindung, die mindestens zwei Aminogruppen aufweist, erfolgen. Das oben gezeigte prinzipielle Reaktionsschema umfassend eine Bindungsknüpfung zwischen einem Stickstoffatom und einer Arylgruppe ist dafür weiterhin gültig.

Bevorzugt wird das erfindungsgemäße Verfahren zur Herstellung von kleinen organischen Verbindungen, d. h. Verbindungen mit einem Molekulargewicht von weniger als 5000 Da, besonders bevorzugt weniger als 3000 Da und ganz besonders bevorzugt weniger als 2000 Da, verwendet.

Bevorzugte Arylaminoverbindungen als Edukte sind Diarylaminoverbindungen. Geeignete Arylaminoverbindungen sind insbesondere auch solche Arylaminoverbindungen, bei denen eine oder beide der Gruppen R bzw. R', welche an das Stickstoffatom gebunden sind, sterisch anspruchsvoll sind. Dabei können auch eine oder beide Gruppen R und/oder R' ein aromatisches oder heteroaromatisches Ringsystem darstellen, welches einen Substituenten in ortho-Position zur Bindung an das Stickstoffatom trägt, oder ein aromatisches oder heteroaromatisches Ringsystem, welches einen ankondensierten Ring in ortho-Position zur Bindung an das Stickstoffatom trägt. Gerade auch mit Arylaminoverbindungen, welche sterisch anspruchsvolle Gruppen R bzw. R' tragen, führt das erfindungsgemäße Verfahren zu sehr guten Ausbeuten, während die Ausbeuten für solche Amine mit NaOtBu als Base wesentlich schlechter sind bzw. je nach Substrat überhaupt kein Umsatz beobachtet wird.

Unter dem Begriff "sterisch anspruchsvoll" wird im Rahmen der vorliegenden Erfindung verstanden, dass eine Gruppe oder ein Substituent eine große räumliche Ausdehnung aufweist. Das Vorhandensein einer sterisch anspruchsvollen Gruppe führt typischerweise zu einer Verlangsamung von Reaktionen in Positionen, welche benachbart oder in räumlicher Nähe zu dieser Gruppe liegen. Im Extremfall ist die Reaktion so stark verlangsamt, dass sie präparativ nicht mehr genutzt werden kann. Sterische Hinderung kann durch beliebige Gruppen verursacht werden und ist umso größer, je größer die Raumausdehnung (sterischer Anspruch) der Gruppe ist. Der sterische Anspruch steigt beispielsweise in der Reihe H, Methyl, Ethyl, Isopropyl, tert-Butyl an, so dass H die am wenigsten sterisch anspruchsvolle Gruppe und tert-Butyl die sterisch anspruchvollste Gruppe in dieser Reihe darstellt.

Geeignete aromatische bzw. heteroaromatische Ringsysteme in der Arylaminoverbindung bzw. geeignete Gruppen R und R' in Formel (1), falls R' nicht für H oder D steht, sind bevorzugt gleich oder verschieden bei jedem Auftreten ausgewählt aus einem aromatischen oder heteroaromatischen Ringsystem mit 5 bis 30 aromatischen Ringatomen, das jeweils durch einen oder mehrere Reste R¹ substituiert sein kann, wobei Reste R und R' miteinander verknüpft sein und so einen Ring bilden können. Besonders bevorzugt stellen R und R', falls R' nicht für H oder D steht, gleich oder verschieden bei jedem Auftreten ein aromatisches oder heteroaromatisches Ringsystem mit 5 bis 18 aromatischen Ringatomen dar, das durch einen oder mehrere Reste R¹ substituiert sein kann.

In einer Ausführungsform der Erfindung stellen R und R', falls R' nicht für H oder D steht, gleich oder verschieden bei jedem Auftreten ein aromatisches oder heteroaromatisches Ringsystem mit 5 bis 18 aromatischen Ringatomen dar, welches mindestens einen Rest R¹ ungleich H und D in ortho-Position zur Bindung an das Stickstoffatom aufweist.

Geeignete aromatische bzw. heteroaromatische Ringsysteme in der Arylaminoverbindung bzw. geeignete Gruppen R und R' in Formel (1), wenn R' nicht für H oder D steht, sind gleich oder verschieden bei jedem Auftreten ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Phenanthren oder Triphenylen, welche jeweils mit einem oder mehreren Resten R¹ substituiert sein können. Weiterhin geeignet sind Arylaminoverbindungen, in denen R und R' zusammen mit dem Stickstoffatom, an das sie gebunden sind, ein Carbazol, Indenocarbazol oder Indolocarbazol bilden, das jeweils durch einen oder mehrere Reste R¹ substituiert sein kann.

Beispiele für geeignete aromatische bzw. heteroaromatische Ringsysteme in der Arylaminoverbindung bzw. für geeignete Gruppen R und R', wenn R' nicht für H oder D steht, sind dabei die Gruppen der folgenden Formeln R-1 bis R-75, 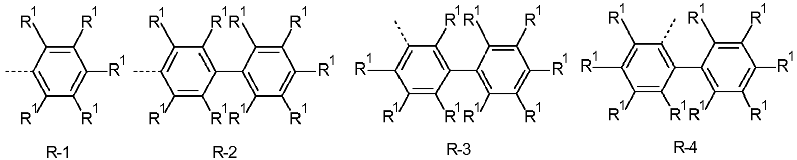 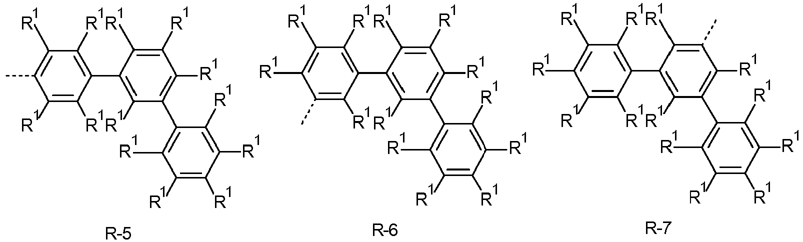 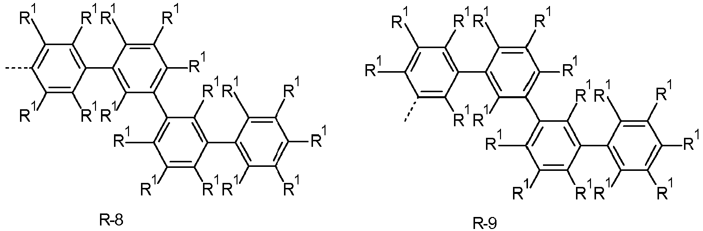 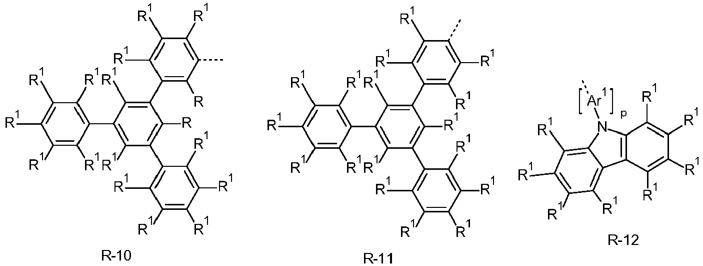 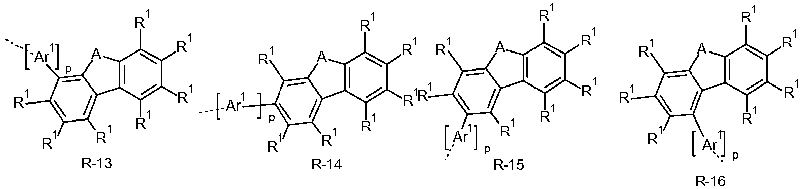 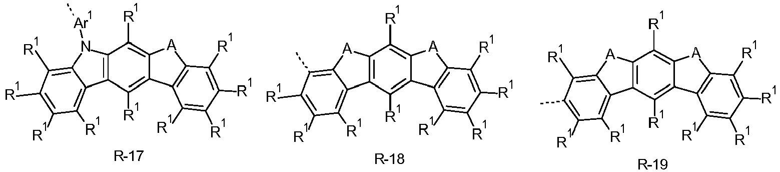 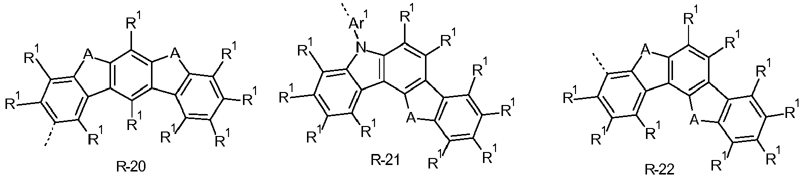 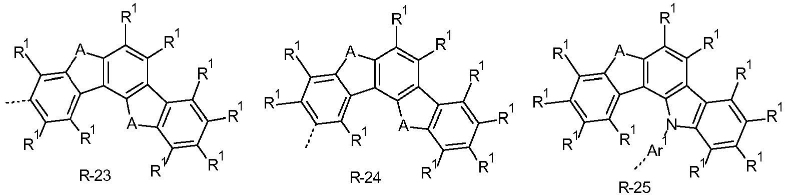 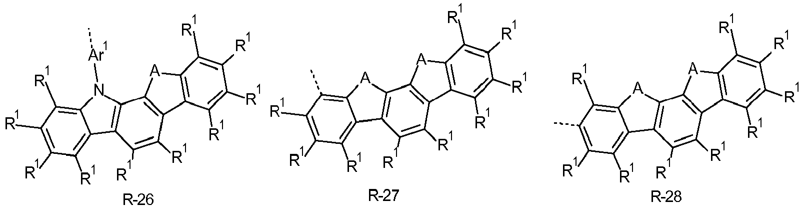 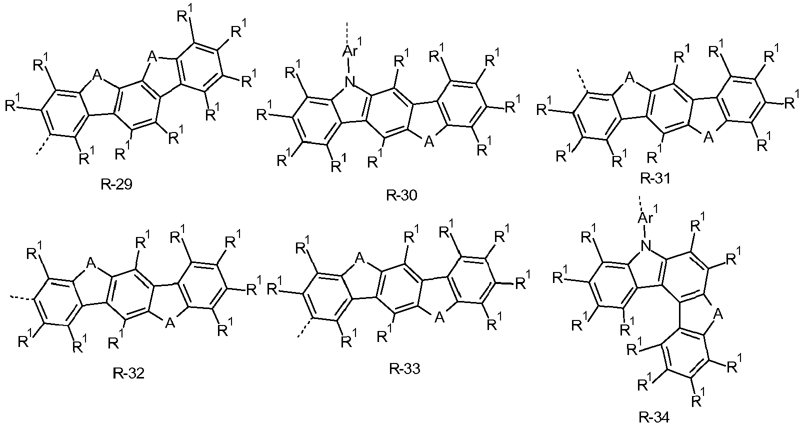 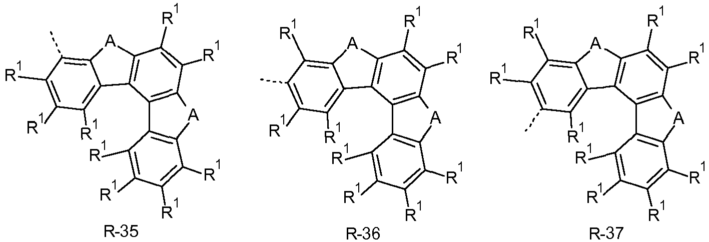 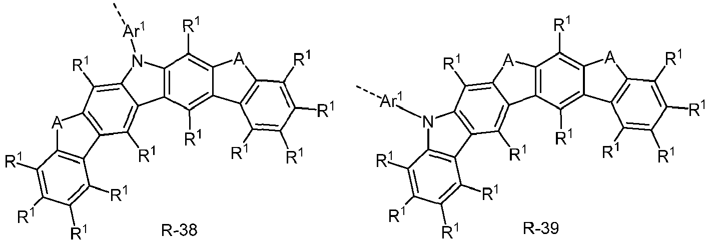 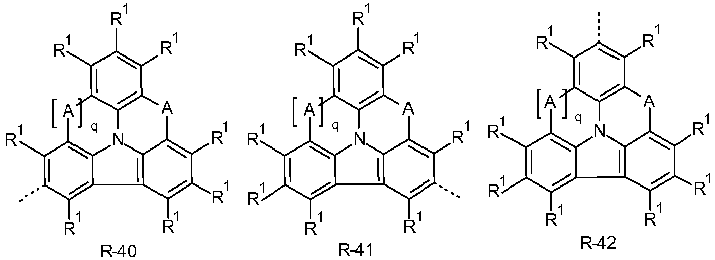 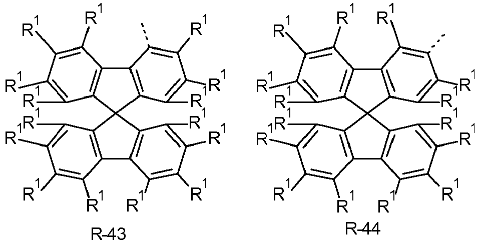 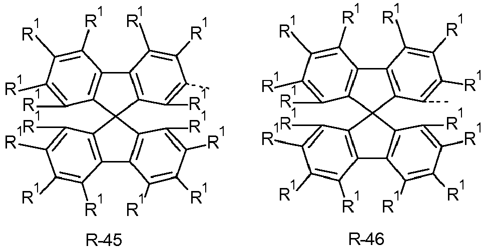 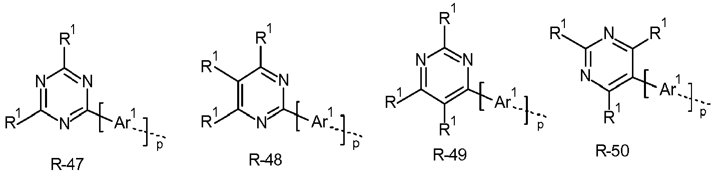 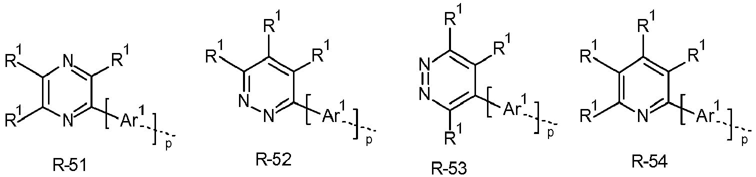 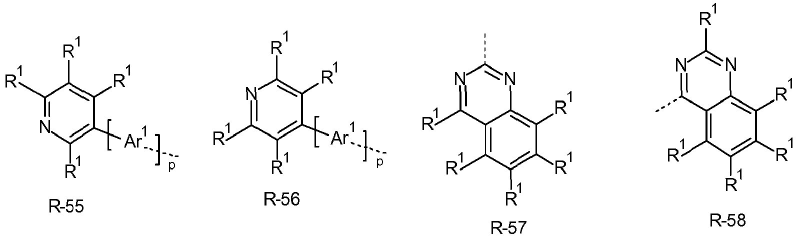 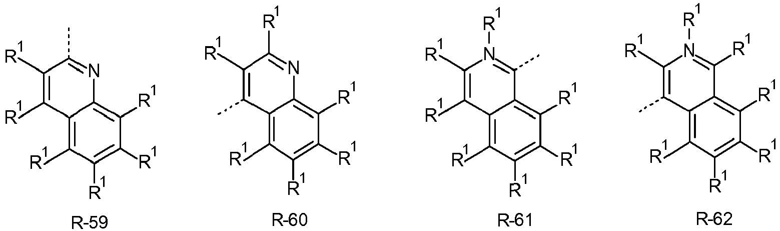 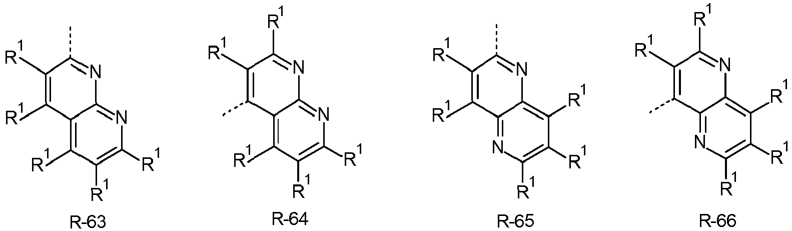 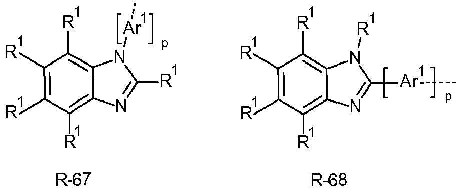 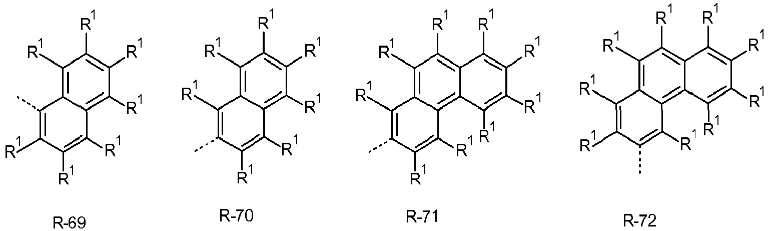 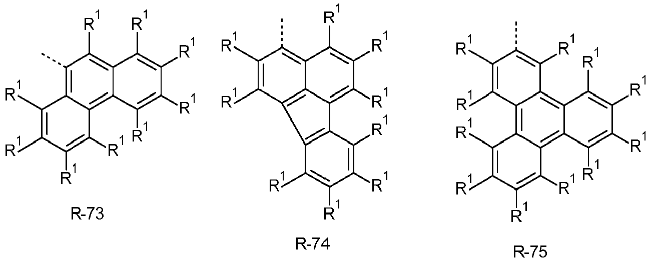 wobei R¹ die oben genannten Bedeutungen aufweist, die gestrichelte Bindung die Bindung an das Stickstoffatom darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an das Stickstoffatom gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Dabei sind die Strukturen R-1 bis R-46 und R-69 bis R-75 bevorzugt.

Bevorzugte Amine sind die in der folgenden Tabelle aufgeführten Verbindungen.

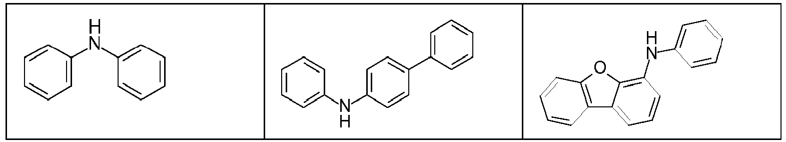

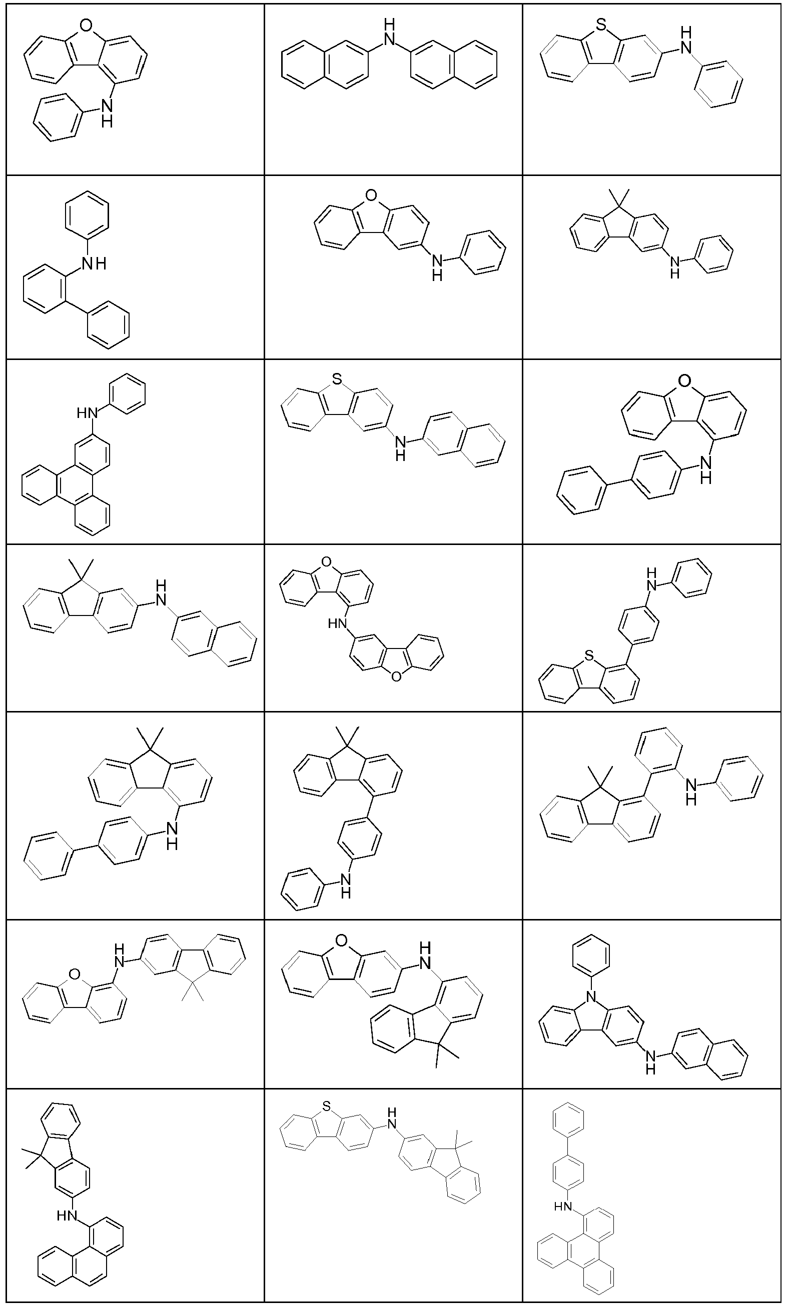

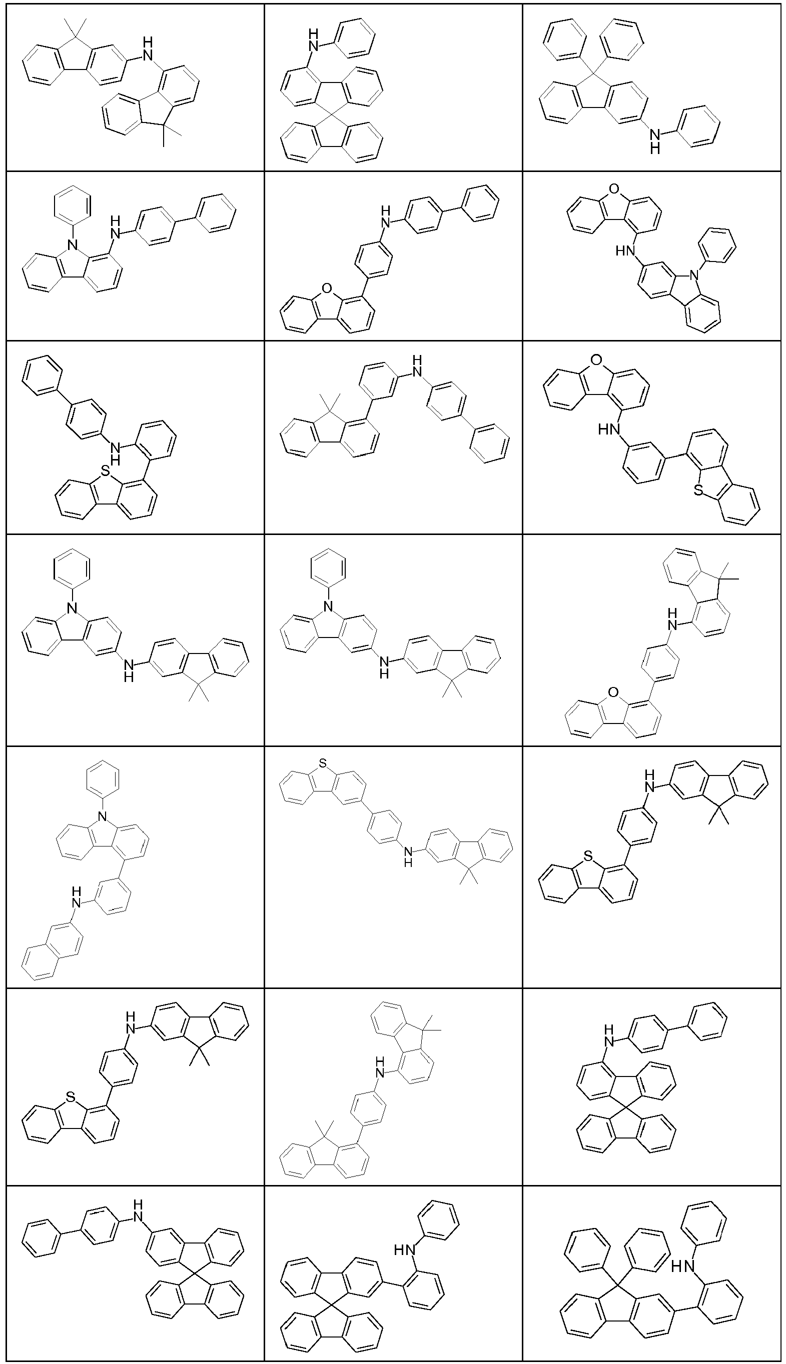

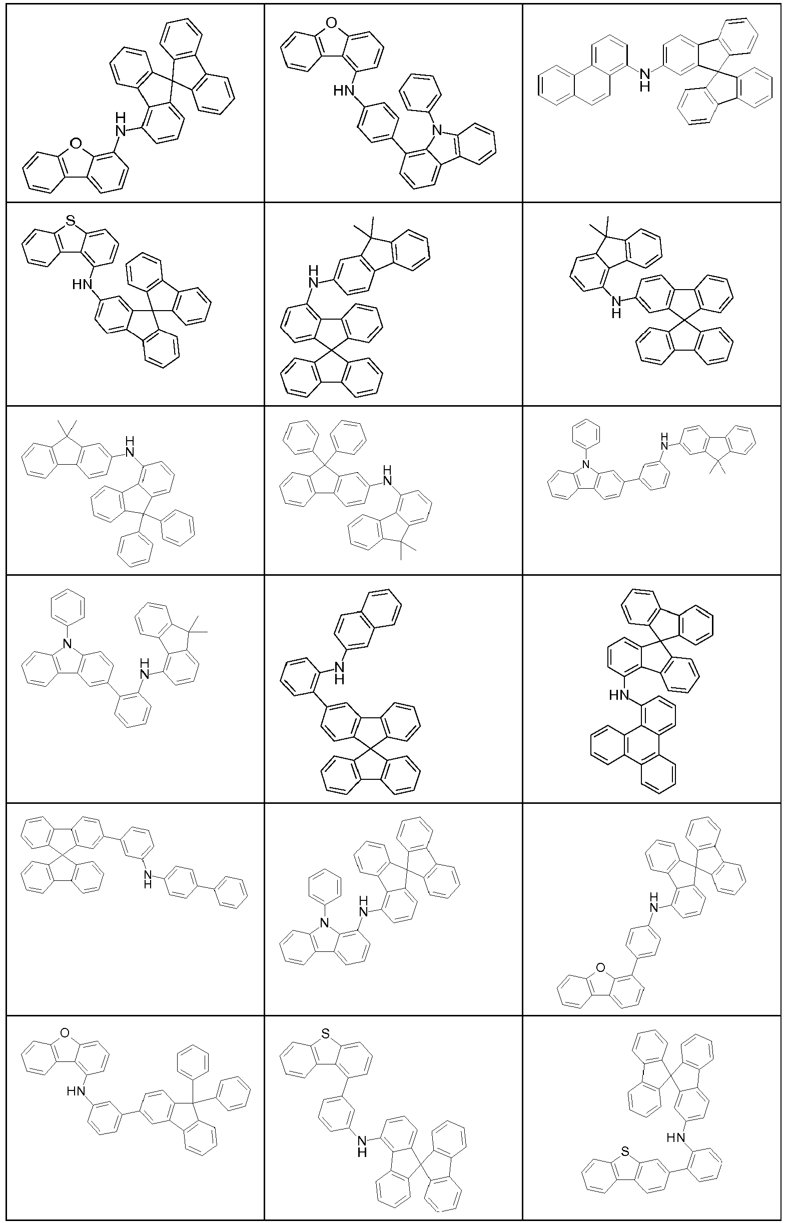

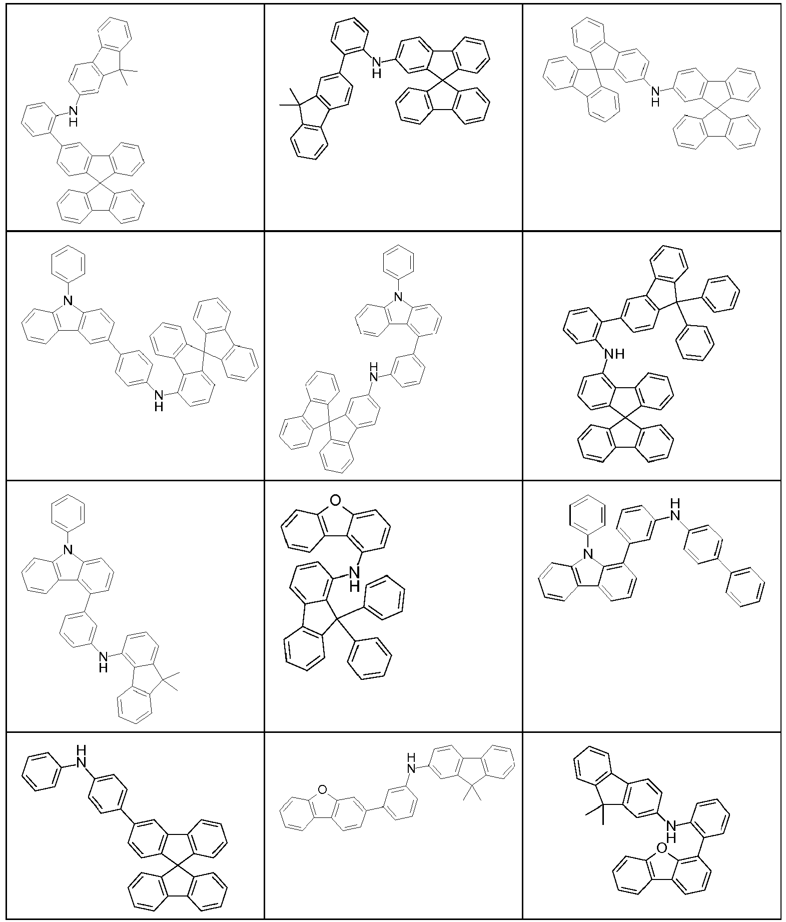

Die Abgangsgruppe in der Arylverbindung bzw. die Gruppe X in der Verbindung der Formel (2), also Ar-(X)ₙ, ist bevorzugt ausgewählt ist aus der Gruppe bestehend aus optional substituiertem Alkylsulfonat, optional substituiertem Arylsulfonat, Halogenid und Diazonium. Beispiele für optional substituierte Alkylsulfonate sind Trifluormethylsulfonat (Triflat, CF₃SO₃-) und Methylsulfonat (Mesylat, CH₃SO₃-). Beispiele für optional substituierte Arylsulfonate sind Phenylsulfonat (C₆H₅-SO₃-) und Tolylsulfonat (Tosylat, CH₃-C₆H₄-SO₃-). Beispiele für Halogenide sind Cl, Br und I. Bevorzugt ist die Abgangsgruppe bzw. die Gruppe X gleich oder verschieden bei jedem Auftreten ausgewählt aus der Gruppe bestehend aus Triflat, Phenylsulfonat und Tosylat, besonders bevorzugt Triflat.

Bevorzugt ist der Index n in der Verbindung der Formel (2) gleich 1, 2, 3, 4 oder 5, besonders bevorzugt gleich 1 oder 2 und ganz besonders bevorzugt gleich 1.

Das aromatische bzw. heteroaromatische Ringsystem in der Arylverbindung bzw. die Gruppe Ar in Formel (2) stellt bevorzugt ein aromatisches oder heteroaromatisches Ringsystem mit 5 bis 30 aromatischen Ringatomen dar, welches mit einem oder mehreren Resten R¹ substituiert sein kann. Dabei ist die Aryl- bzw. Heteroarylgruppe, an die die Abgangsgruppe X gebunden ist, bevorzugt keine elektronenarme Gruppe. Die Gruppe Ar stellt daher bevorzugt ein rein aromatisches Ringsystem oder ein elektronenreiches heteroaromatisches Ringsystem dar. Eine elektronenarme Heteroarylgruppe im Sinne der vorliegenden Erfindung ist eine Sechsring-Heteroarylgruppe, welche mindestens ein Stickstoffatom enthält, oder eine Fünfring-Heteroarylgruppe, welche mindestens zwei Heteroatome enthält, die ausgewählt sind aus N, O oder S, wobei mindestens ein Heteroatom für N steht. Dabei können an diese Heteroarylgruppen jeweils noch weitere Aryl- oder Heteroarylgruppen ankondensiert sein. Dagegen ist eine elektronenreiche Heteroarylgruppe im Sinne der vorliegenden Erfindung eine Fünfring-Heteroarylgruppe, welche genau ein Heteroatom enthält, das ausgewählt ist aus O, S oder N, wobei der Stickstoff substituiert ist und wobei noch weitere Arylgruppen an den Fünfring ankondensiert sein können. Beispiele für elektronenreiche Heteroarylgruppen im Sinne der vorliegenden Erfindung sind Dibenzofuran, Dibenzothiophen, Carbazol, Indenocarbazol und Indolocarbazol.

Geeignete aromatische bzw. heteroaromatische Ringsysteme in der Arylverbindung bzw. geeignete Gruppen Ar in Formel (2) sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Phenanthren oder Triphenylen, welche jeweils mit einem oder mehreren Resten R¹ substituiert sein können.

Weiterhin bevorzugt stellt das aromatische bzw. heteroaromatische Ringsystem in der Arylverbindung bzw. Ar in Formel (2) ein aromatisches oder heteroaromatisches Ringsystem mit 10 bis 30 aromatischen Ringatomen dar, welches mindestens zwei miteinander kondensierte aromatische bzw. heteroaromatische Ringe aufweist, wobei der eine aromatische bzw. heteroaromatische Ring in einer ortho-Position zur Bindung an die Abgangsgruppe bzw. zur Bindung an X an den anderen aromatischen Ring ankondensiert ist. Dies gilt auch beispielsweise für Dibenzofuran oder Dibenzothiophen, welches jeweils in 1-Position eine Abgangsgruppe trägt, oder für Carbazol, welches in 4-Position eine Abgangsgruppe trägt.

Beispiele für geeignete aromatische bzw. heteroaromatische Ringsysteme in der Arylverbindung bzw. für geeignete Gruppen Ar sind dabei die Gruppen der folgenden Formeln Ar-1 bis Ar-53, 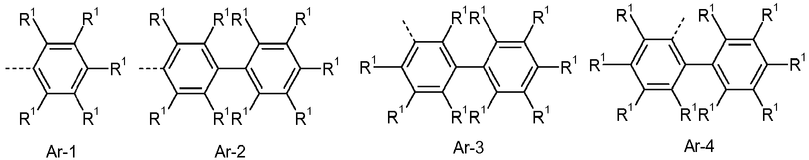 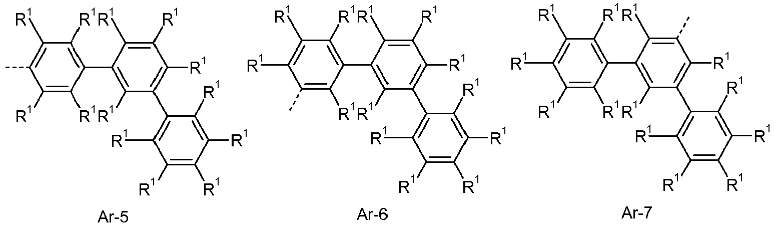 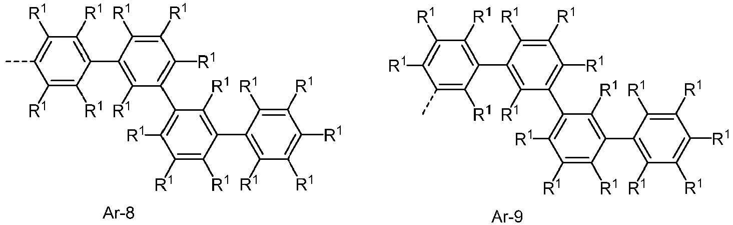 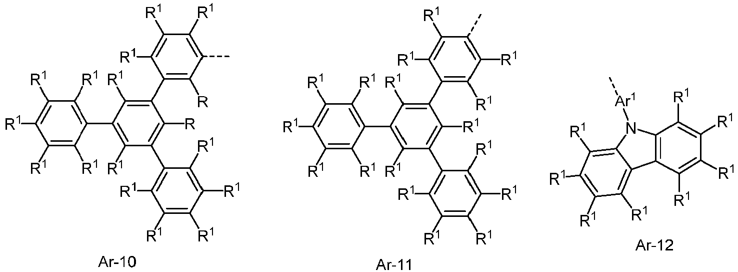 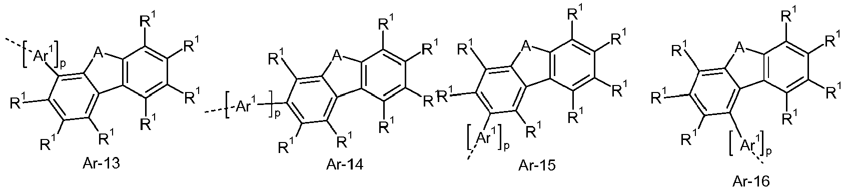 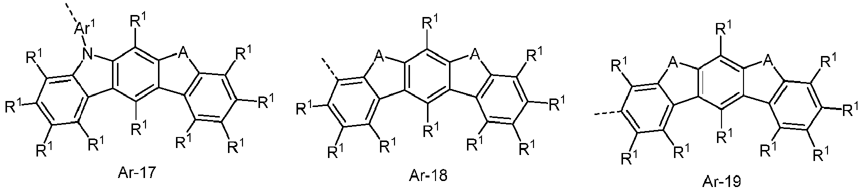 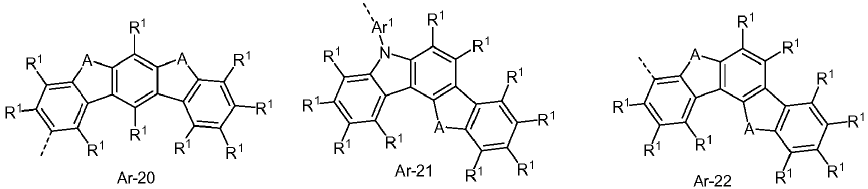 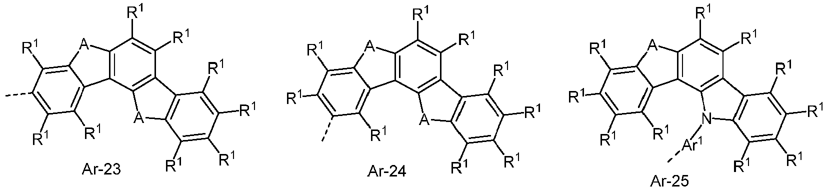 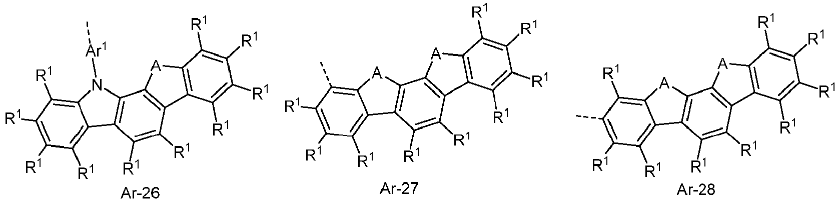 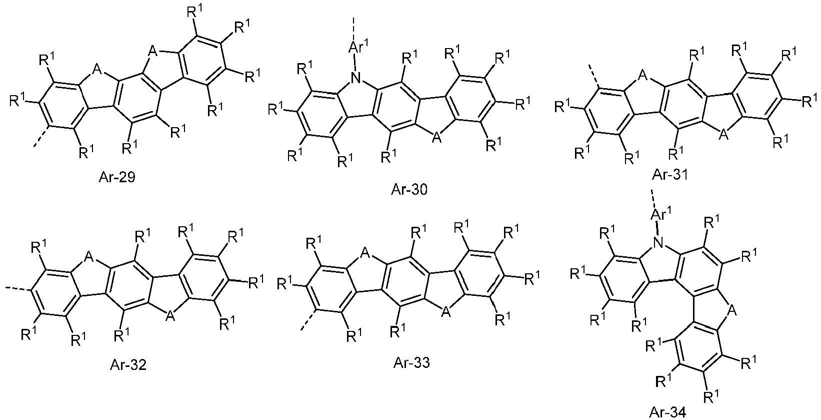 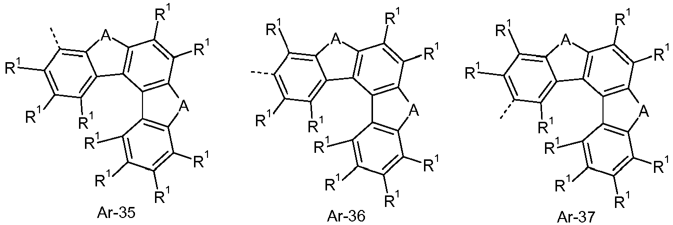 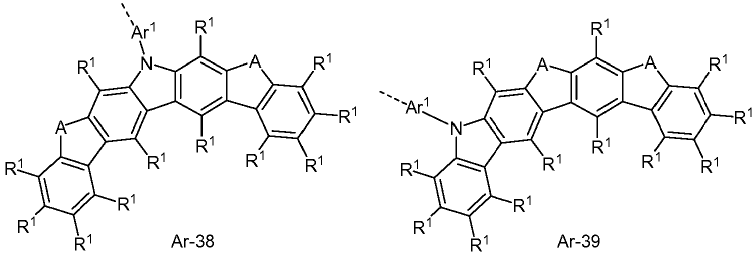 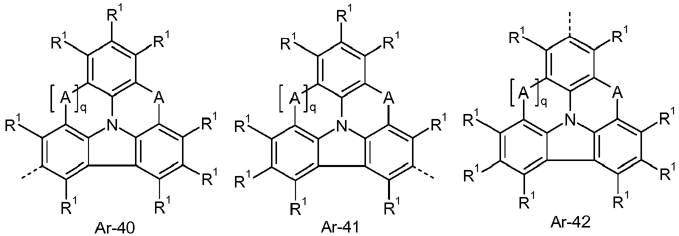 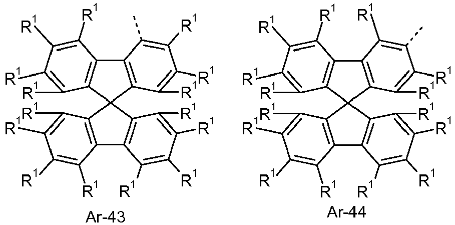 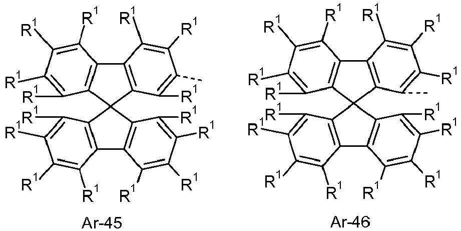 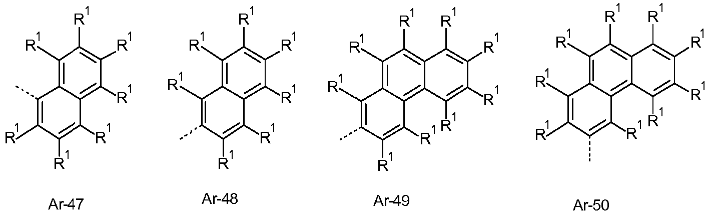 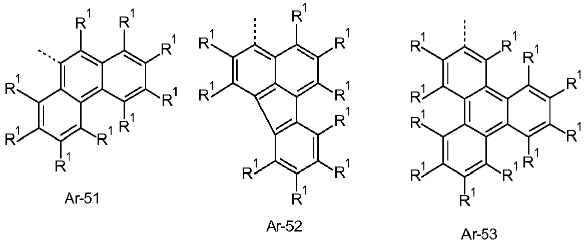 wobei R¹ die oben genannten Bedeutungen aufweist, die gestrichelte Bindung die Bindung an die Abgangsgruppe, also X in der Verbindung Ar-(X)ₙ, darstellt bzw. im Reaktionsprodukt die Bindung an das Stickstoffatom darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an die Abgangsgruppe bzw. die Gruppe X gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Wenn die oben genannten Gruppen für R, R' bzw. Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Dabei können die Arylverbindungen Ar-(X)ₙ auch sterisch anspruchsvolle Gruppen Ar enthalten, ebenso wie Gruppen Ar, welche mit sterisch anspruchsvollen Gruppen R¹, beispielsweise in ortho-Position zur Bindung an die Abgangsgruppe X, substituiert sind, da das erfindungsgemäße Verfahren auch mit sterisch anspruchsvollen Arylverbindungen Ar-(X)ₙ zu guten Ausbeuten führt.

Bevorzugt ist R¹ bei jedem Auftreten gleich oder verschieden ausgewählt aus H, D, F, CN, Si(R²)₃, N(R²)₂ oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten R² substituiert sein können und wobei in den oben genannten Gruppen eine oder mehrere CH₂-Gruppen durch -R²C=CR²- oder -O- ersetzt sein können, oder ein aromatisches oder heteroaromatisches Ringsystem mit 5 bis 24 aromatischen Ringatomen, das jeweils mit einem oder mehreren Resten R² substituiert sein kann; dabei können zwei oder mehr Reste R¹ miteinander verknüpft sein und so einen Ring bilden. Besonders bevorzugt ist R¹ bei jedem Auftreten gleich oder verschieden ausgewählt aus H, D, F, CN, N(R²)₂ oder einer geradkettigen Alkylgruppe mit 1 bis 5 C-Atomen oder einer verzweigten oder cyclischen Alkylgruppe mit 3 bis 6 C-Atomen, wobei die oben genannten Gruppen jeweils mit einem oder mehreren Resten R² substituiert sein können, oder ein aromatisches oder heteroaromatisches Ringsystem mit 6 bis 14 aromatischen Ringatomen, das jeweils mit einem oder mehreren Resten R² substituiert sein kann; dabei können zwei oder mehr Reste R¹ miteinander verknüpft sein und so einen Ring bilden.

Bevorzugt ist R² bei jedem Auftreten gleich oder verschieden ausgewählt aus H, D, F, CN, Si(R³)₃, N(R³)₂ oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -R³C=CR³- oder -O- ersetzt sein können, oder ein aromatisches oder heteroaromatisches Ringsystem mit 5 bis 24 aromatischen Ringatomen, das jeweils mit einem oder mehreren Resten R³ substituiert sein kann; dabei können zwei oder mehr Reste R² miteinander verknüpft sein und so einen Ring bilden. Besonders bevorzugt ist R² bei jedem Auftreten gleich oder verschieden ausgewählt aus H, D, F, CN, N(R³)₂ oder einer geradkettigen Alkylgruppe mit 1 bis 5 C-Atomen oder einer verzweigten oder cyclischen Alkylgruppe mit 3 bis 6 C-Atomen, wobei die oben genannten Gruppen jeweils mit einem oder mehreren Resten R³ substituiert sein können, oder ein aromatisches oder heteroaromatisches Ringsystem mit 6 bis 14 aromatischen Ringatomen, das jeweils mit einem oder mehreren Resten R³ substituiert sein kann; dabei können zwei oder mehr Reste R² miteinander verknüpft sein und so einen Ring bilden.

Bevorzugt ist R³ bei jedem Auftreten gleich oder verschieden H, D, F, eine Alkylgruppe mit 1 bis 5 C-Atomen oder ein aromatisches Ringsystem mit 6 bis 12 aromatischen Ringatomen.

Die als Reaktionsprodukt erhaltenen Verbindungen sind dadurch gekennzeichnet, dass in der Arylverbindung an die Stelle der Bindung zur Abgangsgruppe bzw. die Stelle der Bindung zu X eine Bindung Ar-N tritt, wobei N das Stickstoffatom der Arylaminoverbindung bezeichnet. Bevorzugte Verfahrensprodukte des erfindungsgemäßen Verfahrens sind somit Kombinationen der oben als bevorzugt angegebenen Arylverbindung und Arylaminoverbindung.

Erfindungsgemäß wird die Reaktion in Anwesenheit von Litium-tert-butanolat (LiOtBu) als Base durchgeführt. Die Base wird bevorzugt zu Beginn der Reaktion zur Mischung hinzugegeben.

Die Base wird bevorzugt in einer Menge von 0.5 bis 10 Äquivalenten, bezogen auf die Stoffmenge an eingesetzter Arylverbindung, eingesetzt. Besonders bevorzugt werden 0.6 bis 6 Äquivalente, ganz besonders bevorzugt 1 bis 4 Äquivalente und am stärksten bevorzugt 2 bis 3 Äquivalente an Base eingesetzt. Enthält die Arylverbindung mehr als eine Abgangsgruppe, wird die Base entsprechend der Stoffmenge der Abgangsgruppen in der eingesetzten Arylverbindung eingesetzt.

Im erfindungsgemäßen Verfahren wird eine Palladiumverbindung als Katalysator eingesetzt. Diese kann entweder zu Beginn der Reaktion oder zu einem späteren Zeitpunkt zugegeben werden. Es ist bevorzugt, wenn der Katalysator erst nach abgeschlossener Zugabe der Base zur Mischung hinzugegeben wird. Der Katalysator kann aber auch bereits in der Mischung vorhanden sein, wenn die Base zugegeben wird. Unter dem Begriff "Katalysator" im Rahmen der vorliegenden Anmeldung soll sowohl eine tatsächlich katalytisch aktive Spezies als auch eine Katalysatorvorstufe verstanden werden, welche in der Reaktionsmischung die katalytisch aktive Spezies bildet. Bevorzugte Katalysatoren im Rahmen der vorliegenden Anmeldung sind homogene Katalysatoren, also Katalysatoren, welche im Reaktionsmedium gelöst vorliegen. Als Katalysatoren können generell solche Verbindungen eingesetzt werden, wie sie gemäß dem Stand der Technik für palladium-katalysierte C-N-Kupplungsreaktionen verwendet werden. Diese sind dem Fachmann der organischen Synthese prinzipiell bekannt.

Der Katalysator wird bevorzugt in einer Menge von 0.001 bis 10.0 mol-% eingesetzt, besonders bevorzugt 0.01 bis 5.0 mol-%, ganz besonders bevorzugt 0.1 bis 3.0 mol-%, insbesondere 0.5 bis 2.5 mol-% bezogen auf die Arylverbindung. Enthält die Arylverbindung mehr als eine Abgangsgruppe, wird der Katalysator entsprechend der Stoffmenge der Abgangsgruppen in der eingesetzten Arylverbindung zugegeben.

Der Katalysator umfasst Palladium sowie einen oder mehrere Liganden. Der Katalysator kann als eine einzige Verbindung, umfassend sowohl das Palladium als auch einen oder mehrere Liganden, zugegeben werden. Alternativ kann der Katalysator in situ in der Reaktionsmischung aus separat zugegebener Palladiumverbindung und Ligand gebildet werden.

Geeignete Palladiumverbindungen, die als eigenständige Katalysatoren ohne zusätzliche Liganden eingesetzt werden können, sind ausgewählt aus Pd(PPh₃)₄, Pd-iPr-cinnamyl-Cl CX31 (CAS-Nr. 884879-23-6), Pd-SiPR-cinnamyl-Cl CX32 (CAS-Nr. 884879-24-7), Pd-PEPPSI-iPR (CAS-Nr. 905459-27-0) und Pd-PEPPSI-iPent (CAS-Nr. 1158652-41-5).

Geeignete Palladiumverbindungen als Katalysatorbestandteile, die mit zusätzlichen Liganden eingesetzt werden, sind ausgewählt aus PdCl₂ (CAS-Nr. 7647-10-1), Pd(OAc)₂ (CAS-Nr. 3375-31-3), (CH₃CN)₂PdCl₂ (CAS-Nr. 14592-56-4), Bis(dibenzylidenaceton)palladium (Pd(dba)₂) (CAS-Nr. 32005-36-0) und Tris(dibenzylidenaceton)dipalladium (Pd₂(dba)₃) (CAS-Nr. 51364-51-3). Bevorzugt ist Pd(OAc)₂.

Die Liganden sind bevorzugt ausgewählt aus monodentaten und oligodentaten Liganden, insbesondere monodentaten Liganden. Bevorzugte Liganden sind ausgewählt aus Phosphinen, Phosphiten, Aminen, Aminophosphinen und N-heterocyclischen Carbenen. Geeignete Phosphine und Amine zur Verwendung als Liganden sind in WO 2011/008725, WO 2006/074315 und US 6307087 offenbart. Geeignete N-heterocyclische Carbene zur Verwendung als Liganden sind in CA 2556850 offenbart. Bevorzugt sind monodentate und bidentate Phosphine, insbesondere monodentate Phosphine.

Geeignete Phosphine als Liganden sind ausgewählt aus Dicyclohexylphosphino-2',6'-dimethoxybiphenyl (SPhos, CAS-Nr. 657408-07-6), Dicyclohexylphosphino-2',6'-di-isopropoxybiphenyl (RuPhos, CAS-Nr. 787618-22-8), Di-tert-butyl(2',4',6'-triisopropyl-3,6-dimethoxybiphenyl-2-yl)phosphin (*t*BuBrettPhos, CAS-Nr. 1160861-53-9), Dicyclohexyl(2',4',6'-triisopropyl-3,6-dimethoxybiphenyl-2-yl)phosphin (BrettPhos, CAS-Nr. 1070663-78-3), Trimethylphosphin (CAS-Nr. 594-09-2), Triethylphosphin (CAS-Nr. 554-70-1), Tripropylphosphin (CAS-Nr. 2234-97-1), Triisopropylphosphin (CAS-Nr. 6476-36-4), Tributylphosphin (CAS-Nr. 998-40-3), Tri-tert-butyl-phosphin (CAS-Nr. 13716-12-6), Triphenylphosphin (CAS-Nr. 603-35-0), Di-tert-butyl-chloro-phosphin (CAS-Nr. 13716-10-4), Trimethylphosphit (CAS-Nr.121-45-9), Triethylphosphit (CAS-Nr. 122-52-1), Tripropylphosphit (CAS-Nr. 923-99-9), Triisopropylphosphit (CAS-Nr. 116-17-6), Tributylphosphit (CAS-Nr. 102-85-2), Tricyclohexylphosphit (CAS-Nr. 15205-58-0), Tri(o-tolyl)phosphin (CAS-Nr. 6163-58-2), Triisopropylphosphin (CAS-Nr. 6476-36-4), Tricyclohexylphosphin (CAS-Nr. 2622-14-2), 2,2'-Bis(diphenylphosphino)-1,1'-binaphthyl (BINAP, CAS-Nr. 98327-87-8), 1,2-Bis(dimethylphosphino)ethan (CAS-Nr. 23936-60-9), 1,2-Bis(di-ethylphosphino)ethan (CAS-Nr. 6411-21-8), 1,2-Bis(dipropylphosphino)-ethan (CAS-Nr. 86071-87-6), 1,2-Bis(diisopropylphosphino)ethan (CAS-Nr. 87532-69-2), 1,2-Bis(dibutyl-phosphino)ethan (CAS-Nr. 4141-59-7), 1,2-Bis(dicyclohexylphosphino)ethan (CAS-Nr. 23743-26-2), 1,3-Bis(dicyclo-hexyl-phosphino)propan (CAS-Nr. 103099-52-1), 1,3-Bis(diisopropylphos-phino)propan (CAS-Nr. 91159-11-4), 1,4-Bis(diisopropyl-phosphino)-butan (CAS-Nr. 80499-19-0), 2,4-Bis(dicyclohexylphosphino)pentan (CAS-Nr. 96377-46-7) und 1,1'-Bis(diphenylphosphino)ferroce (dppf, CAS-Nr. 12150-46-8). Weiterhin als Liganden geeignet sind Cy-JohnPhos (CAS-Nr. 247940-06-3), CataCxium Pcy (CAS-Nr. 672937-60-9), Aphos (CAS-Nr. 932710-63-9), XantPhos (CAS-Nr. 161265-03-8) und XPhos (CAS-Nr. 564483-18-7). Bevorzugte Liganden sind SPhos, XPhos und CPhos, besonders bevorzugt SPhos.

Die Palladiumverbindung und der Phosphinligand werden für Monophosphine bevorzugt in einem Verhältnis Pd : Phosphin im Bereich von 1 : 1 bis 1 : 4 eingesetzt, besonders bevorzugt im Bereich von 1 : 1.5 bis 1 : 2.5 und ganz besonders bevorzugt im Bereich von 1 : 1.8 bis 1 : 2.2. Für Biphosphine werden die Palladiumverbindung und der Phosphinligand bevorzugt in einem Verhältnis Pd : Phosphin im Bereich von 1 : 0.5 bis 1 : 2 eingesetzt, besonders bevorzugt im Bereich von 1 : 0.75 bis 1 : 1.5, ganz besonders bevorzugt im Bereich von 1 : 0.9 bis 1 : 1.1.

Das erfindungsgemäße Verfahren wird bevorzugt in flüssiger Phase in Lösung oder Suspension durchgeführt. Dabei fällt je nach Abgangsgruppe das Lithiumsalz, beispielsweise Lithiumtriflat, im Laufe der Reaktion aus der Reaktionsmischung aus. Es kann dabei ein beliebiges aprotisches organisches Lösemittel eingesetzt werden. Geeignet sind die dem Fachmann auf dem Gebiet der organischen Synthese bekannten Lösemittel mit Eignung zur Verwendung in palladiumkatalysierten C-N-Kupplungsreaktionen. Unter Eignung zur Verwendung in den erfindungsgemäßen Reaktionen wird insbesondere verstanden, dass diese inert gegenüber den Reaktionsbedingungen sind. Besonders bevorzugte Lösungsmittel sind ausgewählt aus Benzol, Toluol, 1,2-Xylol, 1,3-Xylol, 1,4-Xylol, Mesitylen, Tetrahydrofuran (THF), 1,4-Dioxan, Dimethoxyethan (dme) und Bis(2-methoxyethyl)ether (diglyme). Bevorzugt werden wasserfreie Lösungsmittel eingesetzt. Ganz besonders bevorzugt sind Toluol, 1,2-Xylol, 1,3-Xylol, 1,4-Xylol und Mesitylen, insbesondere Toluol.

Die Reaktion kann unter Normaldruck oder unter erhöhtem Druck durchgeführt werden.

Gemäß einer bevorzugten Ausführungsform wird die Reaktion unter Inertgasatmosphäre durchgeführt, beispielsweise unter Stickstoff- oder Argonatmosphäre. Besonders gute Ergebnisse werden erzielt, wenn auf die konsequente Einhaltung sehr guter Inertbedingungen geachtet wird, wie sie beispielsweise in einer Glovebox vorliegen.

Die Reaktionsdauer beträgt typischerweise zwischen wenigen Minuten und einigen Tagen, bevorzugt zwischen wenigen Minuten und 100 h, besonders bevorzugt zwischen 15 Minuten und 80 h und ganz besonders bevorzugt zwischen 2 h und 30 h. Die angegebenen Zeiten beziehen sich auf die Gesamtdauer der Reaktion.

Die Reaktionstemperatur beträgt bevorzugt zwischen 0 und 300 °C, besonders bevorzugt zwischen 20 und 200 °C, ganz besonders bevorzugt zwischen 60 und 150 °C. Die angegebenen Reaktionstemperaturen beziehen sich auf die Hauptphase der Reaktion, während der alle Komponenten in der Mischung vorliegen und die C-N-Bindungsknüpfung stattfindet. In einer weiteren bevorzugten Ausführungsform wird die Reaktion unter Rückfluss durchgeführt, so dass sich die Reaktionstemperatur anhand des verwendeten Lösemittels bestimmt. Daher ist eine bevorzugte Reaktionstemperatur bei Verwendung von Toluol als Lösemittel 100 °C, bei 1,2-Xylol 144 °C, bei 1,3-Xylol 139 °C, bei 1,4-Xylol 138 °C und bei Mesitylen 165 °C.

Typischerweise wird die Base zusammen mit den Reaktanden, also der Arylaminoverbindung und der Arylverbindung, in einem Lösemittel vorgelegt und inertisiert. Parallel dazu wird die Katalysatorlösung vorbereitet, wobei bevorzugt dasselbe Lösemittel verwendet wird, welches auch in der Reaktionslösung verwendet wird. Die Katalysatorlösung wird dann zu der Reaktionsmischung zugegeben und die Mischung bei erhöhter Temperatur zur Reaktion gebracht. Alternativ kann der Katalysator bzw. die Palladiumverbindung und der Ligand auch in fester Form zugegeben werden.

Die allgemeine Reaktionsführung und Aufarbeitung sowie die verwendeten Geräte und Reaktionsgefäße sind nicht genauer spezifiziert. Der Fachmann kann unter Beachtung der Ausführungsbeispiele und unter Hinzuziehung seines allgemeinen Fachwissens geeignete Ausführungsformen auswählen. Insbesondere geeignet sind solche Methoden, wie sie dem Fachmann allgemein für Hartwig-Buchwald-Kupplungen bekannt sind.

Wenn es sich bei den im erfindungsgemäßen Verfahren erhaltenen Produkte um sekundäre Arylamine handelt, werden diese bevorzugt als Zwischenstufe zur Herstellung tertiärer Arylamine eingesetzt. Wenn es sich bei den im erfindungsgemäßen Verfahren erhaltenen Produkten um tertiäre Arylamine handelt, werden diese bevorzugt als Funktionsmaterialien in elektronischen Vorrichtungen eingesetzt. Dabei sind die elektronischen Vorrichtungen beispielsweise ausgewählt aus der Gruppe bestehend aus organischen Elektrolumineszenzvorrichtungen (OLEDs), organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs) und organischen Laserdioden (O-Laser). Bevorzugt werden die erhaltenen Verfahrensprodukte als Lochtransportmaterialien und/oder als fluoreszierende Emittermaterialien und/oder als Elektronenblockiermaterialien und/oder als Exzitonenblockiermaterialien und/oder als Matrixmaterialien in elektronischen Vorrichtungen verwendet, insbesondere in organischen Elektrolumineszenzvorrichtungen. Es sind jedoch auch andere Verwendungen möglich, abhängig vom Grundkörper der Produkte und den zusätzlich zur Aminogruppe vorhandenen Substituenten.

Ein weiterer Gegenstand der vorliegenden Erfindung ist die Verwendung von LiOtBu als Base in einer palladiumkatalysierten Kupplungsreaktion zwischen einer Arylaminoverbindung und einer Arylverbindung. Die Arylaminoverbindung und die Arylverbindung sind dabei wie oben angegeben definiert.

Das erfindungsgemäße Verfahren, kombiniert mit einer vorausgehenden Suzuki-Kupplung, ermöglicht die selektive Funktionalisierung aromatischer bzw. heteroaromatischer Verbindungen. So ist es beispielsweise möglich, in einer Arylverbindung, welche mit zwei Abgangsgruppen substituiert ist, von denen eine Abgangsgruppe ein Halogenid und die andere ein Sulfonat ist, zunächst selektiv das Halogenid mit einem Boronsäurederivat in einer Suzuki-Kupplung umzusetzen und dann in einem weiteren Schritt das Sulfonat in einer C-N-Kupplungsreaktion gemäß der vorliegenden Erfindung. Alternativ ist es möglich, zunächst selektiv das Sulfonat mit einem Boronsäurederivat in einer Suzuki-Kupplung umzusetzen und dann in einem weiteren Schritt das Halogenid in einer C-N-Kupplungsreaktion gemäß der vorliegenden Erfindung umzusetzen. Dabei lässt sich über die Wahl des Katalysatorsystems bestimmen, ob selektiv die Sulfonat-Abgangsgruppe oder selektiv die Halogenid-Abgangsgruppe umgesetzt wird. So erhält man eine selektive Umsetzung der Halogenid-Abgangsgruppe, beispielsweise Bromid, in der Suzuki-Kupplung, wenn Tri-ortho-tolylphosphin als Ligand eingesetzt wird, insbesondere in einem Verhältnis Pd : Phosphin in der Größenordnung von 1 : 4. Weiterhin erhält man eine selektive Umsetzung der Sulfonat-Abgangsgruppe, beispielsweise Triflat, in der Suzuki-Kupplung, wenn dppb (1,4-Bis(diphenylphosphino)butan) als Ligand eingesetzt wird, insbesondere in einem Verhältnis Pd : Phosphin in der Größenordnung von 1 : 4. Als weitere Reaktionsbedingungen eignen sich Bedingungen, wie sie üblicherweise für Suzuki-Kupplungen verwendet werden, also beispielsweise der Einsatz von Pd₂(dba)₃ als Palladiumverbindung, Na₂CO₃ oder K₂CO₃ als Base und eine Mischung aus Toluol oder einem anderen organischen Lösemittel und Wasser als Reaktionsmedium.

Das erfindungsgemäße Verfahren weist gegenüber Verfahren gemäß dem Stand der Technik die folgenden Vorteile auf:
1) Die Verwendung von LiOtBu führt im Vergleich zu anderen Basen, insbesondere im Vergleich zu NaOtBu oder KOtBu, zu einer deutlich verbesserten Ausbeute. Dies gilt vor allem beim Einsatz von Sulfonaten, insbesondere von Triflat, als Abgangsgruppe. Das erfindungsgemäße Verfahren zeichnet sich unter anderem dadurch aus, dass eine hohe Ausbeute an Produkt und eine geringe Bildung von Nebenprodukten auftritt.
2) Auch bei der Verwendung sterisch anspruchsvoller Arylaminoverbindungen bzw. sterisch anspruchsvoller Arylverbindungen als Edukte wird im erfindungsgemäßen Verfahren nur wenig Nebenprodukt in Form einer Defunktionalisierung oder einer Kupplung der verwendeten Base beobachtet. Dies stellt eine deutliche Verbesserung gegenüber der Verwendung von NaOtBu oder KOtBu als Base dar.
3) Die Verwendung von LiOtBu als Base führt zu einer hohen Reinheit der erhaltenen Verbindungen. Dies ist gerade für den Einsatz der Verbindungen als Funktionsmaterialien in elektronischen Vorrichtungen von großer Bedeutung. Dabei wird die Bildung von Nebenprodukten, welche sich bereits in geringer Menge abträglich auf die Leistungsdaten der elektronischen Vorrichtung auswirken, durch die Verwendung von LiOtBu zurückgedrängt.
4) Das erfindungsgemäße Verfahren kann auch in einem Maßstab, wie es für die industrielle Anwendung erforderlich ist, also in einem Maßstab von bis zu mehreren Kilogramm durchgeführt werden.
5) Das Verfahren kann mit einer geringen Menge an eingesetztem Katalysator und unter milden Reaktionsbedingungen durchgeführt werden.

Die folgenden Beispiele dienen der Verdeutlichung und detaillierten Beschreibung der Erfindung, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Beispiel 1: Allgemeine Arbeitsvorschrift:

In einer Glovebox werden das Triflat, das Amin und die Base unter einer Argonatmosphäre abgewogen. Nach Zugabe von Katalysator und Ligand wird das Lösungsmittel hinzugefügt und das Gefäß verschlossen. Das Reaktionsgefäß wird aus der Glovebox ausgeschleust und im Ölbad bei 110 °C Innentemperatur für 16 h gerührt. Nach Abkühlen auf Raumtemperatur wird eine Probe entnommen und per HPLC analysiert.

### Beispiel 2: Variation der Base

In der folgenden Reaktion wird Pd-iPr-cinnamyl-Cl CX31 (CAS-Nr. 884879-23-6) als Katalysator eingesetzt. Als Base wird LiOtBu gemäß der vorliegenden Erfindung bzw. als Vergleich gemäß dem Stand der Technik NaOtBu oder KOtBu eingesetzt. Die Ergebnisse sind in Tabelle 1 zusammengefasst. Dabei führt die Reaktion mit LiOtBu als Base zu einer deutlich besseren Ausbeute im Vergleich zu NaOtBu oder KOtBu, und auch bei Reduktion der Katalysatormenge wird weiterhin eine gute Ausbeute erhalten.

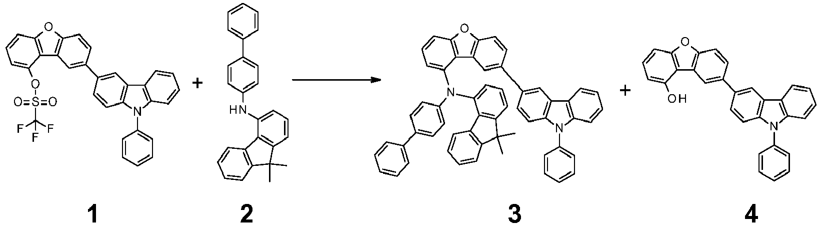

**Tabelle 1^{[a]}: Variation der Base für Pd-iPr-cinnamyl-Cl CX31 als Katalysator**

| **Bsp.** | **[Pd] [mol%]** | **Base (200 mol%)** | **A₁ [%]** | **A₂ [%]** | **A₃ [%]** | **A₄ [%]** |
|---|---|---|---|---|---|---|
| 1 (Vgl.) | 2 | NaOtBu | 0 | 12 | **58** | 25 |
| 2 (Vgl.) | 2 | KOtBu | 0 | 28 | **1** | 52 |
| 3 | 2 | LiOtBu | 1 | 3 | **91** | 1 |
| 4 | 0.5 | LiOtBu | 11 | 17 | **40** | 30 |

| | | | | | | |
|---|---|---|---|---|---|---|
| [a] Reaktionsbedingungen: 0.44 mmol Trifluormethansulfonsäure-8-(9-phenyl-9*H-*carbazol-3-yl)-dibenzofuran-1-yl-ester **1**, 0.48 mmol Biphenyl-4-yl-(9,9- dimethyl-9H-fluoren-4-yl)-amin **2**, 8 mL Toluol, 110 °C, 16 h. Flächenprozente bestimmt durch HPLC. | | | | | | |

### Beispiel 3: Verwendung eines anderen Katalysatorsystems

In diesem Beispiel wird die Reaktion aus Beispiel 2 mit einem anderen Katalysatorsystem unter Variation der Menge an Katalysator und Base durchgeführt. Hierfür wird als Katalysator ein System aus Pd(OAc)₂ und SPhos verwendet (Tabelle 2).

**Tabelle 2: Optimierung der Versuchsparameter für das Pd(OAc)₂ / SPhos / LiOtBu System.^{[a]}**

| **Bsp.** | **Pd(OAc)₂ [mol%]** | **SPhos [mol%]** | **LiOtBu [mol%]** | **A₁ [%]** | **A₂ [%]** | **A₃ [%]** | **A₄ [%]** |
|---|---|---|---|---|---|---|---|
| 1 | 1 | 2 | 220 | 2 | 6 | **82** | 8 |
| 2 | 1 | 2 | 150 | 8 | 3 | **75** | 8 |
| 3 | 1 | 2 | 110 | 25 | 3 | **57** | 12 |
| 4 | 0.5 | 1 | 220 | 2 | 8 | **74** | 14 |
| 5 | 1 | 1 | 220 | 2 | 6 | **83** | 5 |
| 6 | 1 | 1.5 | 220 | 1 | 8 | **82** | 6 |
| 7 | 2 | 4 | 200 | 0 | 3 | **94** | 0 |
| 8 | 2 | 4 | 250 | 0 | 3 | **95** | 0 |
| 9 | 2 | 4 | 300 | 0 | 3 | **95** | 0 |

| | | | | | | | |
|---|---|---|---|---|---|---|---|
| [a] Reaktionsbedingungen: 0.44 mmol Trifluormethansulfonsäure-8-(9-phenyl-9H-carbazol-3-yl)-dibenzofuran-1-yl-ester **1**, 0.48 mmol Biphenyl-4-yl-(9,9- dimethyl-9H-fluoren-4-yl)-amin **2**, 8 mL Toluol, 110 °C, 16 h. Flächenprozente bestimmt durch HPLC. | | | | | | | |

### Beispiel 4: Verwendung unterschiedlicher Liganden und unterschiedlicher Substrate

In diesem Beispiel wird die Reaktion mit anderen Liganden und anderen Substraten unter Beibehaltung von Pd(OAc)₂ als Palladiumquelle und LiOtBu als Base durchgeführt (Tabellen 3 und 4).

### a) Umsetzung eines Dibenzofurantriflats mit Bis(para-biphenyl)amin

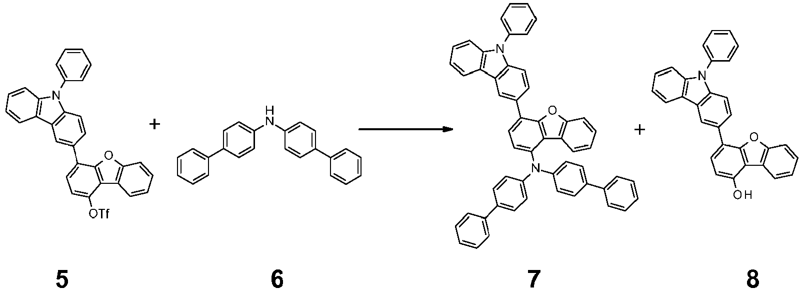

**Tabelle 3^{[8]}: Variation des Lianden**

| **Bsp.** | **Ligand** | **A₅ [%]** | **A₆ [%]** | **A₇ [%]** | **A₈ [%]** |
|---|---|---|---|---|---|
| 1 | SPhos 4 mol% | 0.02 | 0.69 | **96.4** | k.A. |
| 2 | XantPhos 2 mol% | 0.09 | 4.79 | **71.3** | k.A. |
| 3 | CPhos 4 mol% | 0.54 | 1.09 | **95.8** | k.A. |
| 4 | XPhos 4 mol% | 0.32 | 0.76 | **96.9** | k.A. |

| | | | | | |
|---|---|---|---|---|---|
| [a] Reaktionsbedingungen: 0.60 mmol Trifluormethansulfonsäure-4-(9-phenyl-9H-carbazol-3-yl)-dibenzofuran-1-yl-ester **5**, 0.60 mmol Bis-biphenyl-4-yl-amin **6**, 250 mol% LiOtBu, 2 mol% Pd(OAc)₂, 6.0 ml Toluol, 110 °C, 16 h. Flächenprozent bestimmt durch HPLC. | | | | | |

### b) Umsetzung eines Dibenzofurantriflats mit einem para-Biphenyl-4-fluorenyl-amin

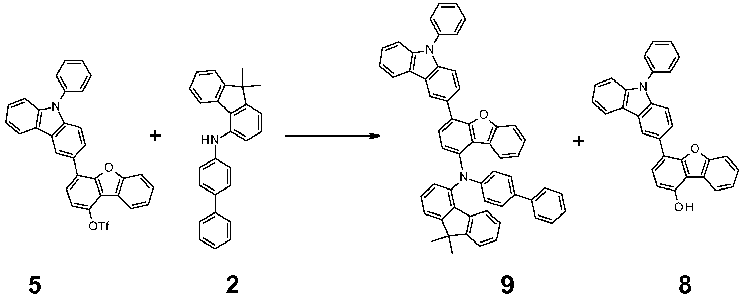

**Tabelle 4^{[8]}: Variation der Liganden**

| **Bsp.** | **Ligand** | **A₅ [%]** | **A₂ [%]** | **A₉ [%]** | **A₈ [%] k.A.** | |
|---|---|---|---|---|---|---|
| 1 | SPhos | 1.37 | 4.71 | **84.0** | | |
| 2 | XPhos | 0.75 | 2.98 | **87.5** | k.A. | |

| | | | | | | |
|---|---|---|---|---|---|---|
| [a] Reaktionsbedingungen: 0.35 mmol Trifluormethansulfonsäure-4-(9-phenyl-9*H-*carbazol-3-yl)-dibenzofuran-1-yl-ester **5**, 0.39 mmol Biphenyl-4-yl-(9,9-dimethyl-9*H-*fluoren-4-yl)-amin **2,** 220 mol% LiO*t*Bu, 2 mol% Pd(OAc)₂, 4 mol% Ligand, 6.0 ml Toluol, 110 °C, 16 h. Flächenprozent bestimmt durch HPLC. | | | | | | |

### Beispiel 5: Verwendung unterschiedlicher Substrate

In diesem Beispiel werden die Reaktionsbedingungen aus Tabelle 3, Eintrag 4 und Tabelle 4, Eintrag 2 auf andere Substrate übertragen (Tabelle 5). Dafür wird in diesem Beispiel Phenyltriflat mit ortho-Biphenyl-phenyl-amin als sterisch anspruchsvollem sekundären Amin umgesetzt. Dabei führt die Reaktion mit LiOtBu als Base zu einer signifikant besseren Ausbeute im Vergleich zu NaOtBu.

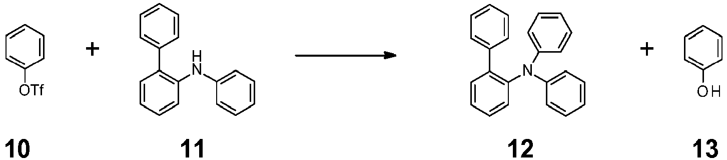

**Tabelle 5^{[a]}:**

| **Bsp.** | **Base** | **A₁₀ [%]** | **A₁₁ [%]** | **A₁₂ [%]** | **A₁₃ [%]** |
|---|---|---|---|---|---|
| 1 | LiOtBu | 0 | 15.3 | **76.4** | **k.A.** |
| 2 (Vgl.) | NaOtBu | 0 | 91.3 | **2.3** | k.A. |

| | | | | | |
|---|---|---|---|---|---|
| [a] Reaktionsbedingungen: 0.86 mmol Phenyl-trifluormethansulfonat **10**, 0.95 mmol Biphenyl-2-yl-phenylamin **11,** 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 6: Umsetzung von Phenyltriflat mit Carbazol

In diesem Beispiel wird als sekundäres Amin Carbazol eingesetzt, also ein Amin, in dem die beiden aromatischen Gruppen miteinander verknüpft sind (Tabelle 6). Dabei führt die Reaktion mit LiOtBu als Base zu einer deutlich besseren Ausbeute im Vergleich zu NaOtBu.

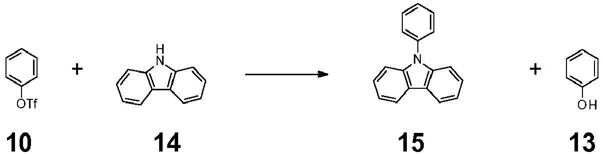

**Tabelle 6^{[a]}:**

| **Bsp.** | **Base** | **A₁₀ [%]** | **A₁₄ [%]** | **A₁₅ [%]** | **A₁₃ [%]** |
|---|---|---|---|---|---|
| 1 | LiO*t*Bu | 0 | k.A. | **96.1** | k.A. |
| 2 (Vgl.) | NaOtBu | 0 | k.A. | **77.8** | k.A. |

| | | | | | |
|---|---|---|---|---|---|
| [a] Reaktionsbedingungen: 0.86 mmol Phenyl-trifluormethansulfonat **10**, 0.95 mmol Carbazol **14**, 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 7: Umsetzung von 1-Naphthyltriflat mit sekundärem Amin

In diesem Beispiel wird als Arylverbindung 1-Naphthyltriflat eingesetzt, also eine Verbindung mit einer kondensierten Arylgruppe (Tabelle 7). Dabei führt die Reaktion mit LiOtBu als Base zu einer signifikant besseren Ausbeute im Vergleich zu NaOtBu.

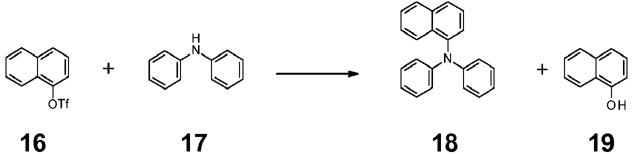

**Tabelle 7 ^{[a]}:**

| **Bsp.** | **Base** | **A₁₆ [%]** | **A₁₇ [%]** | **A₁₈ [%]** | **A₁₉ [%]** |
|---|---|---|---|---|---|
| 1 | LiO*t*Bu | 0.5 | 3.2 | **94.1** | 0.2 |
| 2 (Vgl.) | NaO*t*Bu | 0 | 32.4 | **8.5** | 46.9 |

| | | | | | |
|---|---|---|---|---|---|
| [a] Reaktionsbedingungen: 0.70 mmol 1-Naphthyl-trifluormethansulfonat **16,** 0.77 mmol Diphenylamin **17**, 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 8: Umsetzung von 1-Naphthyltriflat mit sterisch anspruchsvollem sekundärem Amin

In diesem Beispiel wird als Arylverbindung 1-Naphthyltriflat, also eine Arylverbindung mit kondensierter Arylgruppe, mit Phenyl-ortho-biphenylamin als sterisch anspruchsvollem sekundären Amin umgesetzt (Tabelle 8). Dabei führt die Reaktion mit LiOtBu als Base zu einer guten Ausbeute, während mit NaOtBu als Base fast überhaupt keine Umsetzung zum tertiären Amin beobachtet wird.

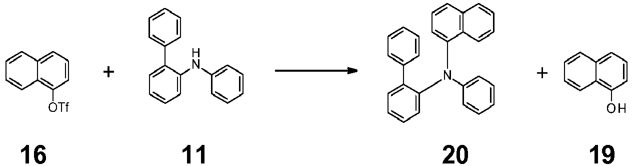

**Tabelle 8 ^{[a]}:**

| **Bsp.** | **Base** | **A₁₆ [%]** | **A₁₁ [%]** | **A₂₀ [%]** | **A₁₉ [%]** |
|---|---|---|---|---|---|
| 1 | LiO*t*Bu | 19.9 | 21.4 | **51.6** | 0 |
| 2 (Vgl.) | NaO*t*Bu | 0.1 | 55.9 | **0.4** | 35.6 |

| | | | | | |
|---|---|---|---|---|---|
| [a] Reaktionsbedingungen: 0.70 mmol 1-Naphthyl-trifluormethansulfonat **16**, 0.77 mmol Biphenyl-2-yl-phenylamin **11**, 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 9: Umsetzung von sterisch anspruchsvollem Triflat und sterisch anspruchsvollem sekundären Amin

In diesem Beispiel wird ortho-Biphenyltriflat als sterisch anspruchsvolle Arylverbindung mit Phenyl-ortho-biphenylamin als sterisch anspruchsvollem Amin umgesetzt (Tabelle 9). Dabei führt die Reaktion mit LiOtBu als Base zu einer guten Ausbeute, während mit NaOtBu als Base überhaupt keine Umsetzung zum tertiären Amin beobachtet wird.

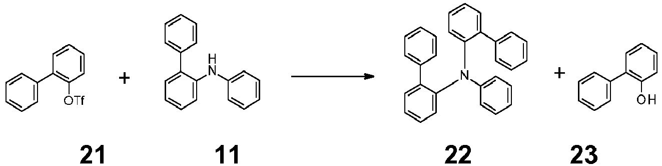

**Tabelle 9^{[a]}:**

| **Bsp.** | **Base** | **A₂₁ [%]** | **A₁₁ [%]** | **A₂₂ [%]** | **A₂₃ [%]** |
|---|---|---|---|---|---|
| 1 | LiOtBu | 3.3 | 29.9 | **21.2** | **2.5** |
| 2 (Vgl.) | NaOtBu | 0 | 63.6 | **0** | 33.7 |

| | | | | | |
|---|---|---|---|---|---|
| [a] Reaktionsbedingungen: 0.65 mmol Biphenyl-2-yl-trifluormethansulfonat **21**, 0.72 mmol Biphenyl-2-yl-phenylamin **11**, 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 10: Umsetzung von sterisch anspruchsvollem Triflat mit sekundärem Amin

In diesem Beispiel wird ortho-Biphenyltriflat als sterisch anspruchsvolle Arylverbindung mit Diphenylamin als sekundäres Amin umgesetzt (Tabelle 10). Dabei führt die Reaktion mit LiOtBu als Base zu einer sehr guten Ausbeute, während mit NaOtBu als Base überhaupt keine Umsetzung zum tertiären Amin beobachtet wird.

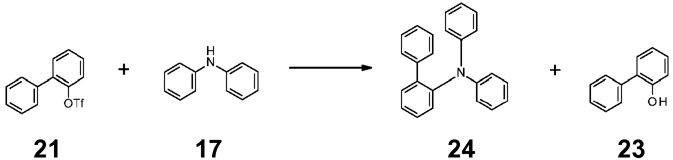

**Tabelle 10^{[a]}:**

| **Bsp.** | **Base** | **A₂₁ [%]** | **A₁₇ [%]** | **A₂₄ [%]** | **A₂₃ [%]** |
|---|---|---|---|---|---|
| 1 | LiOtBu | 0 | 5.5 | 89.7 | 0.3 |
| 2 (Vgl.) | NaOtBu | 0 | 45.5 | 0 | 52.5 |

| | | | | | |
|---|---|---|---|---|---|
| [a] Reaktionsbedingungen: 0.65 mmol Biphenyl-2-yl-trifluormethansulfonat **21,** 0.72 mmol Diphenylamin **17,** 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 11: Umsetzung von sterisch anspruchsvollem 9-Phenyl-9H-carbazol-4-yl-trifluormethansulfonsäureester mit Carbazol

In diesem Beispiel wird ein Carbazolylderivat, welches in 4-Position mit Triflat als Abgangsgruppe substituiert ist, als sterisch anspruchsvolle Arylverbindung mit Carbazol als sekundärem Amin umgesetzt (Tabelle 11). Dabei führt die Reaktion mit LiOtBu als Base zu einer guten Ausbeute, während mit NaOtBu als Base überhaupt keine Umsetzung zum C-N-gekuppelten Produkt beobachtet wird.

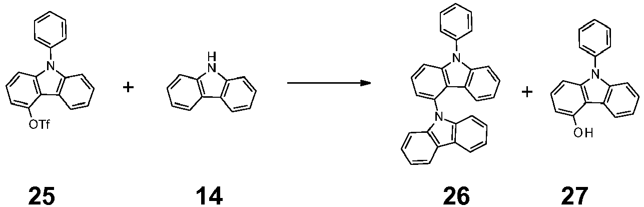

**Tabelle 11^{[a]}:**

| **Bsp.** | **Base** | **A₂₅ [%]** | **A₁₄ [%]** | **A₂₆ [%]** | **A₂₇ [%]** |
|---|---|---|---|---|---|
| 1 | LiOtBu | 61.5 | 13.5 | **18.1** | 4.1 |
| 2 (Vgl.) | NaOtBu | 0 | 92.4 | **0** | 2.0 |

| | | | | | |
|---|---|---|---|---|---|
| [a] Reaktionsbedingungen: 0.50 mmol 9-Phenyl-9*H*-carbazol-4-yl-trifluormethansulfon-säureester **25**, 0.55 mmol Carbazol **14**, 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 12: Umsetzung von elektronenreichem Triflat mit sterisch anspruchsvollem sekundären Amin

In diesem Beispiel wird para-Methoxyphenyl-triflat als Arylverbindung mit para-Biphenyl-2,4-(diphenyl)phenyl-amin als sterisch anspruchsvollem sekundären Amin umgesetzt (Tabelle 12). Dabei führt die Reaktion mit LiOtBu als Base zu einer guten Ausbeute, während mit NaOtBu als Base fast keine Umsetzung zum tertiären Amin beobachtet wird.

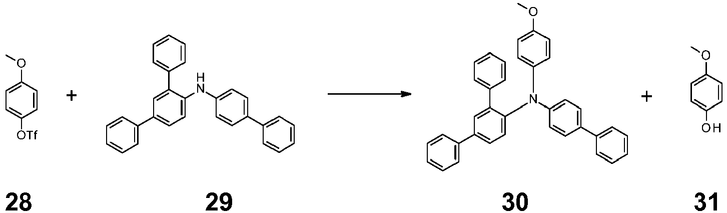

**Tabelle 12^{[a]}:**

| **Bsp.** | **Base** | **A₂₈ [%]** | **A₂₉ [%]** | **A₃₀ [%]** | **A₃₁ [%]** |
|---|---|---|---|---|---|
| 1 | LiOtBu | 3.7 | 59.5 | **28.2** | k.A. |
| 2 (Vgl.) | NaOtBu | 0 | 95.3 | **< 1.0** | k.A. |

| | | | | | |
|---|---|---|---|---|---|
| [a] Reaktionsbedingungen: 0.76 mmol 4-Methoxyphenyl-trifluormethansulfonat **28**, 0.83 mmol 2,4-Diphenyl-N-(4-phenylphenyl)anilin **29**, 220 mol% Base, 2 mol% Pd(OAc)₂, 4 mol% XPhos, 5.7 ml Toluol, 110 °C, 16 h. Flächenprozente bestimmt durch HPLC. | | | | | |

### Beispiel 13: Selektivität der Umsetzung von Chloraryltriflat mit sterisch anspruchsvollem sekundären Amin

In diesem Beispiel wird als Arylverbindung para-Chlorphenyl-triflat eingesetzt, welches mit der Chlorgruppe und der Triflatgruppe zwei potentielle Abgangsgruppen aufweist. Dieses wird mit ortho-Biphenyl-phenyl-amin als sterisch anspruchsvollem Amin selektiv an der Triflatgruppe umgesetzt (Tabelle 13). Dabei führt die Reaktion mit LiOtBu als Base zu einer guten Ausbeute, während mit NaOtBu als Base fast keine Umsetzung zum tertiären Amin beobachtet wird.

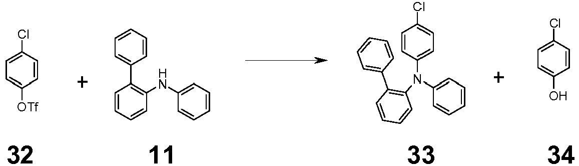

**Tabelle 13^{[a]}:**

| **Bsp.** | **Base** | **A₃₂ [%]** | **A₁₁ [%]** | **A₃₃ [%]** | **A₃₄ [%] k.A.** |
|---|---|---|---|---|---|
| 1 | LiOtBu | 1.7 | 20.3 | **71.3** | |
| 2 (Vgl.) | NaOtBu | 1.5 | 85.6 | **5** | k.A. |

| | | | | | |
|---|---|---|---|---|---|
| [a] Reaktionsbedingungen: 0.76 mmol 4-Chlorphenyl-trifluormethansulfonat **32**, 0.84 mmol Biphenyl-2-yl-phenyl-amin **11**, 220 mol% Base, 2 mol% Pd(OAc)₂, 4 mol% XPhos, 5.7 ml Toluol, 110 °C, 15 h. Flächenprozente bestimmt durch HPLC. | | | | | |

### Beispiel 14: Umsetzung von Phenyltriflat mit einem primärem Amin zu einem sekundären Amin

In diesem Beispiel wird Phenyltriflat mit Anilin als primäre Arylaminoverbindung umgesetzt (Tabelle 14). Dabei führt die Reaktion mit LiOtBu als Base zu einer deutlich besseren Ausbeute im Vergleich zu NaOtBu.

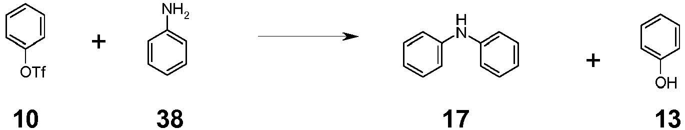

**Tabelle 14^{[a]}:**

| **Bsp.** | **Base** | **A₁₀ [%]** | **A₃₈ [%]** | **A₁₇ [%]** | **A₁₃ [%] k.A.** |
|---|---|---|---|---|---|
| 1 | LiO*t*Bu | 0 | 1.2 | 98.8 | |
| 2 (Vgl.) | NaOtBu | 0 | 17.2 | 82.8 | k.A. |

| | | | | | |
|---|---|---|---|---|---|
| [a] Reaktionsbedingungen: 0.88 mmol Phenyl-trifluormethansulfonat **10**, 0.97 mmol Anilin **38**, 220 mol% Base, 2 mol% Pd(OAc)₂, 4 mol% XPhos, 5.7 ml Toluol, 110 °C, 15 h. Flächenprozente bestimmt durch HPLC. | | | | | |

### Beispiel 15: Umsetzung von Phenylsulfonat mit sterisch anspruchsvollem sekundärem Amin

In diesem Beispiel wird ein Phenylsulfonat als Abgangsgruppe verwendet. Hierfür wird para-Tolyl-phenylsulfonat mit ortho-Biphenyl-phenyl-amin als sterisch anspruchsvolles sekundäres Amin umgesetzt (Tabelle 15). Dabei führt die Reaktion mit LiOtBu als Base zu einer deutlich besseren Ausbeute im Vergleich zu NaOtBu.

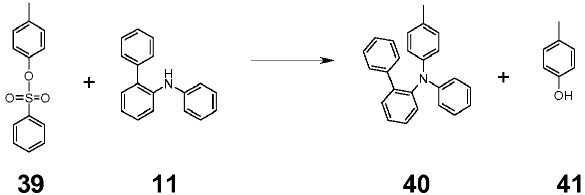

**Tabelle 15^{[a]}:**

| **Bsp.** | **Ligand** | **Base** | **A₃₉ [%]** | **A₁₁ [%]** | **A₄₀ [%]** | **A₄₁ [%] k.A.** |
|---|---|---|---|---|---|---|
| 1 | XPhos | LiO*t*Bu | 7.4 | 52.4 | **32.7** | |
| 2 (Vgl.) | XPhos | NaOtBu | 3.12 | 81.3 | **10.9** | k.A. |
| 3 | BrettPhos | LiO*t*Bu | 3.6 | 68.3 | **23.6** | k.A. |
| 4 (Vgl.) | BrettPhos | NaOtBu | 0 | 92.4 | **4** | k.A. |

| | | | | | | |
|---|---|---|---|---|---|---|
| [a] Reaktionsbedingungen: 0.81 mmol Phenyl 4-methylbenzolsulfonat **39**, 0.88 mmol Biphenyl-2-yl-phenyl-amin **11,** 220 mol% Base, 2 mol% Pd(OAc)₂, 4 mol% Ligand, 5.7 ml Toluol, 110 °C, 15 h. Flächenprozente bestimmt durch HPLC. | | | | | | |

### Beispiel 16: Umsetzung im größeren Maßstab

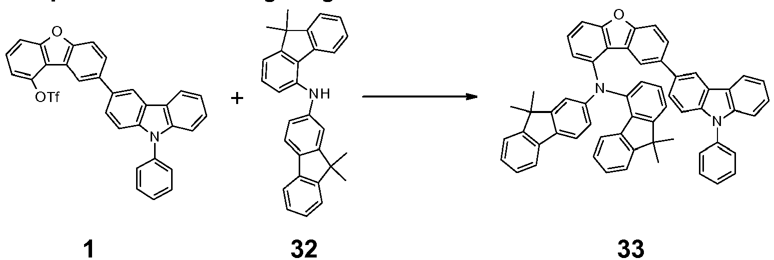

In einem Autoklaven werden 98.0 g (172 mmol) Trifluormethansulfonsäure-8-(9-phenyl-9*H*-carbazol-3-yl)-dibenzofuran-1-yl-ester **1**, 76.1 g (189 mmol, 110 mol%) (9,9-Dimethyl-9*H*-fluoren-4-yl)-(9,9-dimethyl-9*H*-fluoren-2-yl)-amin **32** sowie 30.3 g (379 mmol, 220 mol%) LiO*t*Bu eingewogen und in 1.90 L wasserfreiem Toluol suspendiert. Das Gemisch wird dreimal entgast und mit Stickstoff geflutet. Parallel werden in einer Glovebox unter Argonatmosphäre 0.78 g (3.49 mmol, 2.03 mol%) Pd(OAc)₂ und 3.37 g (7.07 mmol, 4.11 mol%) XPhos abgewogen, in 100 ml wasserfreiem Toluol gelöst und 1 h gerührt. Anschließend wird die Katalystaorlösung im Stickstoff-Gegenstrom zu der Eduktlösung gegeben und die Mischung für 16 h bei 110 °C gerührt. Nach Abkühlen auf Raumtemperatur wird eine 3%ige *N*-Acetylcystein Lösung zugegeben und das Gemisch 30 min gerührt. Nach Phasentrennung wird die organische Phase über basisches Aluminiumoxid filtriert und anschließend aufkonzentriert. Der Rückstand wird mit einem Gemisch aus *n*-Heptan und Ethanol versetzt, und der kristallisierte Feststoff wird durch zwei Heißextraktionen mit *n*-Heptan / Toluol über basischem Aluminiumoxid weiter aufgereinigt. Anschließend wird der erhaltene Feststoff in THF gelöst und über basisches Aluminiumoxid filtriert. Das Filtrat wird eingeengt und mit n-Heptan versetzt. Der erhaltene Feststoff wird abschließend im Hochvakuum sublimiert. Ausbeute: 85.2 g (105 mmol, 65.0 %), Reinheit > 99.9 % nach HPLC.

### Beispiel 17: Brom-selektive Suzuki-Reaktion

Dieses Beispiel beschreibt die Herstellung des Edukts für Beispiel 16, welches eine Triflat-Abgangsgruppe aufweist, aus einer Verbindung, die sowohl Triflat, als auch Brom als Abgangsgruppen aufweist. Dabei wird selektiv die Bromgruppe in der Suzuki-Kupplung umgesetzt.

### Allgemeine Arbeitsvorschrift:

Das Edukt und die Boronsäure werden zusammen mit dem Katalysator und dem Liganden eingewogen und im Lösungsmittel gelöst. Das Gemisch wird 5 min. lang mit Argon gesättigt und anschließend bei der angegebenen Temperatur für 16 h gerührt. Nach Abkühlen auf Raumtemperatur wird eine Probe entnommen und per HPLC oder GC-MS analysiert. Die Ergebnisse des Katalysator-Screenings sind in Tabelle 16 zusammengefasst.

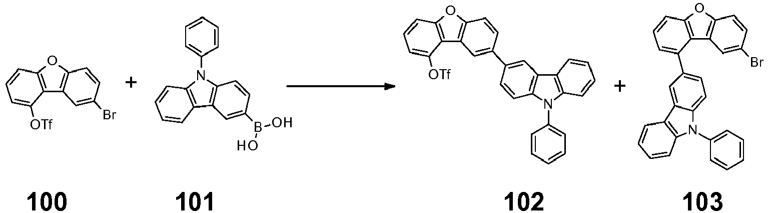

**Tabelle 16: Katalysator Screening für die Darstellung von 102.^{[a]}**

| **Bsp.** | **Katalysatorsystem (Verhältnis)** | **A₁₀₀ [%]** | **A₁₀₂ [%]** | **A₁₀₃ [%]** |
|---|---|---|---|---|
| 1 | Pd(PPh₃)₄ | 2 | **52** | 33 |
| 2 | Pd₂dba₃ / PPh₃ (1:2) | 2 | **52** | 31 |
| 3 | Pd₂dba₃ / P(o-Tol)3 (1:2) | 0 | **80** | 3 |
| 4 | Pd₂dba₃ / SPhos (1:2) | 5 | **11** | 73 |
| 5 | Pd₂dba₃ / XPhos (1:2) | 4 | **24** | 61 |
| 6 | Pd₂dba₃ / CMPhos (1:2) | 18 | **6** | 5 |
| 7 | Pd₂dba₃ / PCy₃ (1:2) | 3 | **70** | 11 |
| 8 | Pd₂dba₃ / P(t-Bu)₃ (1:2) | 3 | **7** | 1 |
| 9 | Pd₂dba₃ / CataCxium A (1:2) | 0 | **74** | 9 |
| 10 | Pd-PEPPSI-iPr | 0 | **73** | 12 |

| | | | | |
|---|---|---|---|---|
| [a] Reaktionsbedingungen: 1.27 mmol Trifluormethansulfonsäure-8-brom-dibenzofuran-1-yl-ester **100,** 1.52 mmol 9-Phenylcarbazol-3-yl-boronsäure, 2.53 mmol Kaliumcarbonat, 1 mol% [Pd], 5.75 ml Toluol, 2.00 ml Wasser, 90 °C, 16 h. Flächenprozent bestimmt durch HPLC. Gehalt der Boronsäure kann wegen Co-Eluation mit Toluol nicht bestimmt werden. | | | | |

Die Versuchsparamter für das Katalysator/Ligand System Pd₂dba₃ / P(*o*-Tol)₃ werden weiter optimiert (Tabelle 17).

**Tabelle 17: Optimierung der Versuchsparameter für das System Pd₂dba₃ / P(o-Tol)₃ System zur Darstellung von 102.^{[a]}**

| **Bsp.** | **mol% Pd₂dba₃ / P(*o*-Tol)₃** | **K₂CO₃ [mol%]** | **101 [mol%]** | **T [°C]** | **A₁₀₀ [%]** | **A₁₀₂ [%]** | **A₁₀₃ [%]** |
|---|---|---|---|---|---|---|---|
| 1 | 0.25 : 1 | 200 | 120 | 80 | 0 | **78** | 0 |
| 2 | 0.25 : 1.5 | 200 | 120 | 80 | 0 | **79** | 0 |
| 3 | 0.5:2 | 200 | 110 | 80 | 0 | **83** | 0 |
| 4 | 0.5:2 | 120 | 110 | 80 | 0 | **83** | 0 |
| 5 | 0.5:2 | 120 | 100 | 70 | 2 | **83** | 0 |
| 6 | 0.5:2 | 120 | 80 | 70 | 5 | **79** | 0 |
| 7[b] | 0.5:2 | 200 | 120 | 80 | 0 | **87 (78^{[c]})** | 0 |

| | | | | | | | |
|---|---|---|---|---|---|---|---|
| [a] Reaktionsbedingungen: 1.27 mmol mmol ruormetansuonsure-rom-dibenzofuran-1-yl-ester **100**, 5.75 ml Toluol, 2.00 ml Wasser, 16 h. Flächenprozente bestimmt durch HPLC. Gehalt der Boronsäure konnte wegen Co-Eluation mit Toluol nicht bestimmt werden, [b] Up-Scaling Reaktion mit 78 g **100**. [c] Isolierte Ausbeute. | | | | | | | |

Analog zu den Reaktionsbedingungen in Tabelle 17 können folgende Verbindungen dargestellt werden:

| **Edukt 1** | **Edukt 2** | **Produkt** | **Produktgehalt nach GC-MS** |
|---|---|---|---|
| 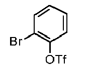 | 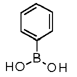 | 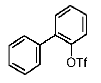 | 81 % |
| 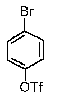 | 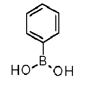 | 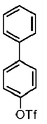 | 78 % |
| 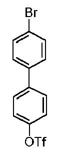 | 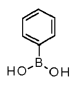 | 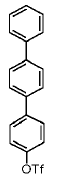 | 95 % |
| 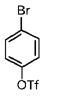 | 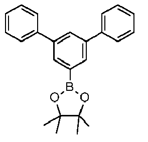 | 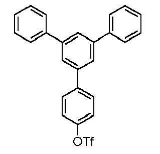 | 90 % |

### Beispiel 18: Triflat-selektive Suzuki-Reaktion

Dieses Beispiel beschreibt die Herstellung von Edukten für das erfindungsgemäße Verfahren, welche eine Brom-Abgangsgruppe aufweisen, aus einer Verbindung, die sowohl Triflat, als auch Brom als Abgangsgruppen aufweist. Dabei wird selektiv die Triflatgruppe in der Suzuki-Kupplung umgesetzt.

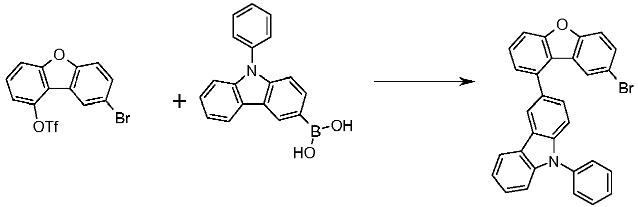

Es werden 20.0 g (49.2 mmol) Trifluormethansulfonsäure-8-brom-dibenzofuran-1-yl-ester, 14.8 g (51.6 mmol) *N*-Phenylcarbazol-3-boronsäure und 13.6 g (98.3 mmol) Kaliumcarbonat in 400 ml Toluol und 100 ml Wasser suspendiert und 20 min lang mit Argon inertisiert. Nach Zugabe von 337 mg (0.37 mmol) Pd₂dba₃ und 629 mg (1.47 mmol) dppb wird das Gemisch für 48 h zum Rückfluss erhitzt. Nach Abkühlen auf Raumtemperatur wird die wässrige Phase abgetrennt, die organische Phase wird zweimal mit Wasser gewaschen, über Natriumsulfat getrocknet und eingeengt. Der Rückstand wird aus 500 ml Ethanol kristallisiert und als Feststoff erhalten. Ausbeute: 22.0 g (45.1 mmol, 92 %). Reinheit > 98 %.

## Patentansprüche

1. Verfahren zur Herstellung einer sekundären bzw. tertiären Arylaminoverbindung durch eine palladiumkatalysierte Kupplungsreaktion zwischen einer primären bzw. sekundären Arylaminoverbindung und einer Arylverbindung, **dadurch gekennzeichnet, dass** das Verfahren in Anwesenheit von Lithium-tert-butanolat (LiOtBu) als Base durchgeführt wird.

2. Verfahren nach Anspruch 1, **dadurch gekennzeichnet, dass** als Arylaminoverbindung eine Verbindung gemäß Formel (1) eingesetzt wird, 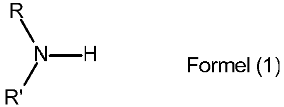 wobei für die verwendeten Symbole gilt:
R ist gleich oder verschieden bei jedem Auftreten ein aromatisches oder heteroaromatisches Ringsystem mit 5 bis 60 aromatischen Ringatomen, das jeweils durch einen oder mehrere Reste R¹ substituiert sein kann;
R' ist gleich oder verschieden R oder steht für H oder D; dabei können R und R' direkt oder über eine Gruppe R¹ miteinander verknüpft sein und so zusammen mit dem Stickstoffatom, an das sie binden, ein Ringsystem bilden;
R¹ ist gleich oder verschieden bei jedem Auftreten ausgewählt aus H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 5 bis 30 aromatischen Ringatomen, das jeweils durch einen oder mehrere Reste R² substituiert sein kann, oder einer Aryl- oder Heteroaryloxygruppe mit 5 bis 30 aromatischen Ringatomen, die durch einen oder mehrere Reste R² substituiert sein kann; dabei können zwei oder mehr Reste R¹ miteinander verknüpft sein und so einen Ring bilden;
R² ist gleich oder verschieden bei jedem Auftreten ausgewählt aus H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 5 bis 30 aromatischen Ringatomen, das jeweils durch einen oder mehrere Reste R³ substituiert sein kann, oder einer Aryl- oder Heteroaryloxygruppe mit 5 bis 30 aromatischen Ringatomen, die durch einen oder mehrere Reste R³ substituiert sein kann; dabei können zwei oder mehr Reste R² miteinander verknüpft sein und so einen Ring bilden;
R³ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und so einen Ring bilden.

3. Verfahren nach Anspruch 1 oder 2, **dadurch gekennzeichnet, dass** als Arylverbindung eine Verbindung gemäß Formel (2) eingesetzt wird, 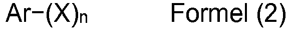 wobei R¹, R² und R³ die in Anspruch 2 aufgeführten Bedeutungen aufweisen und für die weiteren verwendeten Symbole und Indizes gilt:
Ar ist ein aromatisches oder heteroaromatisches Ringsystem mit 5 bis 60 aromatischen Ringatomen, das mit einem oder mehreren Resten R¹ substituiert sein kann;
X ist bei jedem Auftreten gleich oder verschieden eine Abgangsgruppe;
n ist eine ganze Zahl von 1 bis 10.

4. Verfahren nach Anspruch 2 oder 3, **dadurch gekennzeichnet, dass** R und R', wenn R' nicht für H oder D steht, gleich oder verschieden bei jedem Auftreten ausgewählt sind aus der Gruppe bestehend aus Phenyl, Biphenyl, Terphenyl, Quaterphenyl, Fluorenyl, Spirobifluorenyl, Naphthyl, Indolyl, Benzofuranyl, Benzothienyl, Carbazolyl, Dibenzofuranyl, Dibenzothienyl, Indenocarbazolyl, Indolocarbazolyl, Phenanthryl oder Triphenylenyl, welche jeweils mit einem oder mehreren Resten R¹ substituiert sein können.

5. Verfahren nach einem oder mehreren der Ansprüche 1 bis 4, **dadurch gekennzeichnet, dass** die Arylverbindung mit einer Abgangsgruppe substituiert ist und die Abgangsgruppe bzw. die Gruppe X in der Verbindung der Formel (2) ausgewählt ist aus der Gruppe bestehend aus optional substituiertem Alkylsulfonat, optional substituiertem Arylsulfonat, Halogenid und Diazonium.

6. Verfahren nach Anspruch 5, **dadurch gekennzeichnet, dass** die Arylverbindung mit einer Abgangsgruppe substituiert ist und die Abgangsgruppe bzw. die Gruppe X in der Verbindung der Formel (2) ausgewählt ist aus der Gruppe bestehend aus Triflat, Phenylsulfonat und Tosylat.

7. Verfahren nach einem oder mehreren der Ansprüche 1 bis 6, **dadurch gekennzeichnet, dass** das aromatische bzw. heteroaromatische Ringsystem in der Arylverbindung bzw. die Gruppe Ar in der Verbindung der Formel (2) ausgewählt ist aus der Gruppe bestehend aus Phenyl, Biphenyl, Terphenyl, Quaterphenyl, Fluorenyl, Spirobifluorenyl, Naphthyl, Indolyl, Benzofuranyl, Benzothienyl, Carbazolyl, Dibenzofuranyl, Dibenzothienyl, Indenocarbazolyl, Indolocarbazolyl, Phenanthrenyl oder Triphenylenyl, welche jeweils mit einem oder mehreren Resten R¹ substituiert sein können.

8. Verfahren nach einem oder mehreren der Ansprüche 1 bis 7, **dadurch gekennzeichnet, dass** Lithium-tert-butanolat in einer Menge von 0.5 bis 10 Äquivalenten, bezogen auf die Stoffmenge an eingesetzter Arylverbindung, eingesetzt wird.

9. Verfahren nach einem oder mehreren der Ansprüche 1 bis 8, **dadurch gekennzeichnet, dass** als Katalysator PdCl₂, Pd(OAc)₂, (CH₃CN)₂PdCl₂, Bis(dibenzylidenaceton)dipalladium oder Tris(dibenzylidenaceton)dipalladium zusammen mit mindestens einem Liganden eingesetzt wird.

10. Verfahren nach einem oder mehreren der Ansprüche 1 bis 9, **dadurch gekennzeichnet, dass** als Ligand für den Katalysator Phosphine, Phosphite, Amine, Aminophosphine oder N-heterocyclischen Carbene eingesetzt werden.

11. Verfahren nach Anspruch 10, **dadurch gekennzeichnet, dass** das Phosphin ausgewählt ist aus der Gruppe bestehend aus Dicyclohexylphosphino-2',6'-dimethoxybiphenyl, Dicyclohexylphosphino-2',6'-di-iso-propoxybiphenyl, Di-tert-butyl(2',4',6'-triisopropyl-3,6-dimethoxybi-phenyl-2-yl)phosphin, Dicyclohexyl(2',4',6'-triisopropyl-3,6-dimethoxy-biphenyl-2-yl)phosphin, Trimethylphosphin, Triethylphosphin, Tripropylphosphin, Triisopropylphosphin, Tributylphosphin, Tri-tert-butyl-phosphin, Tricyclohexylphosphin, Triphenylphosphin, Di-tert-butyl-chloro-phosphin, Triphenylphosphin, Tri(o-tolyl)phosphin, Triisopropylphosphin, Tricyclohexylphosphin, 2,2'-Bis(diphenylphosphino)-1,1'-bi-naphthyl (BINAP), 1,2-Bis(dimethylphosphino)ethan, 1,2-Bis(diethyl-phosphino)ethan, 1,2-Bis(dipropylphosphino)-ethan, 1,2- Bis(diiso-propylphosphino)ethan, 1,2-Bis(dibutyl-phosphino)ethan, 1,2- Bis(di-cyclohexylphosphino)ethan, 1,3-Bis(dicyclohexyl-phosphino)propan, 1,3-Bis(diisopropylphosphino)propan, 1,4-Bis(diisopropyl-phosphino)-butan, 2,4-Bis(dicyclohexylphosphino)pentan, 1,1'-Bis(diphenyl-phosphino)ferrocen, SPhos, PCy₃, Cy-JohnPhos, CataCxium Pcy, APhos, XantPhos, dppf, XPhos und BrettPhos.

12. Verfahren nach einem oder mehreren der Ansprüche 9 bis 11, **dadurch gekennzeichnet, dass** die Palladiumverbindung und der Phosphinligand für Monophosphine in einem Verhältnis Pd : Phosphin von 1 : 1 bis 1 : 4 eingesetzt werden und dass die Palladiumverbindung und der Phosphinligand für Biphosphine in einem Verhältnis Pd : Phosphin von 1 : 0.5 bis 1 : 2 eingesetzt werden.

13. Verfahren nach einem oder mehreren der Ansprüche 1 bis 12, **dadurch gekennzeichnet, dass** das Verfahren in einem oder mehreren aprotischen organischen Lösemitteln, bevorzugt ausgewählt aus Benzol, Toluol, 1,2-Xylol, 1,3-Xylol, 1,4-Xylol, Mesitylen, Tetrahydrofuran (THF), 1,4-Dioxan, Dimethoxyethan (dme) und Bis(2-methoxyethyl)ether (diglyme), durchgeführt wird.

14. Verfahren nach einem oder mehreren der Ansprüche 1 bis 13, **dadurch gekennzeichnet, dass** das Verfahren unter Inertgasatmosphäre durchgeführt wird.

15. Verwendung von LiOtBu als Base in einer palladiumkatalysierten Kupplungsreaktion zwischen einer Arylaminoverbindung und einer Arylverbindung.

## Claims

1. Process for the preparation of a secondary or tertiary arylamino compound by a palladium-catalysed coupling reaction between a primary or secondary arylamino compound and an aryl compound, **characterised in that** the process is carried out in the presence of lithium tert-butoxide (LiOtBu) as base.

2. Process according to Claim 1, **characterised in that** the arylamino compound employed is a compound of the formula (1), 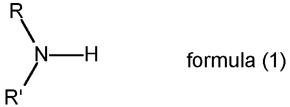 where the following applies to the symbols used:
R is, identically or differently on each occurrence, an aromatic or heteroaromatic ring system having 5 to 60 aromatic ring atoms, which may in each case be substituted by one or more radicals R¹;
R' is, identically or differently, R or stands for H or D; R and R' may be linked to one another directly or via a group R¹ and thus form a ring system together with the nitrogen atom to which they are bonded;
R¹ is selected, identically or differently on each occurrence, from H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R² and where one or more CH₂ groups in the above-mentioned groups may be replaced by -R²C=CR²-, -C=C-, Si(R²)₂, C=O, C=S, C=NR², -C(=O)O-, -C(=O)NR²-, NR², P(=O)(R²), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R², or an aryl- or heteroaryloxy group having 5 to 30 aromatic ring atoms, which may be substituted by one or more radicals R²; two or more radicals R¹ may be linked to one another and thus form a ring;
R² is selected, identically or differently on each occurrence, from H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C=C-, Si(R³)₂, C=O, C=S, C=NR³, -C(=O)O-, -C(=O)NR³⁻, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R³, or an aryl- or heteroaryloxy group having 5 to 30 aromatic ring atoms, which may be substituted by one or more radicals R³; two or more radicals R² may be linked to one another and thus form a ring;
R³ is on each occurrence, identically or differently, H, D, F or an aliphatic, aromatic or heteroaromatic organic radical having 1 to 20 C atoms, in which, in addition, one or more H atoms may be replaced by D or F; two or more substituents R³ may be linked to one another and thus form a ring.

3. Process according to Claim 1 or 2, **characterised in that** the aryl compound employed is a compound of the formula (2), 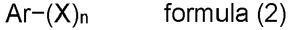 where R¹, R² and R³ have the meanings given in Claim 2 and the following applies to the other symbols and indices used:
Ar is an aromatic or heteroaromatic ring system having 5 to 60 aromatic ring atoms, which may be substituted by one or more radicals R¹;
X is on each occurrence, identically or differently, a leaving group;
n is an integer from 1 to 10.

4. Process according to Claim 2 or 3, **characterised in that** R and R', if R' does not stand for H or D, are selected, identically or differently on each occurrence, from the group consisting of phenyl, biphenyl, terphenyl, quaterphenyl, fluorenyl, spirobifluorenyl, naphthyl, indolyl, benzofuranyl, benzothienyl, carbazolyl, dibenzofuranyl, dibenzothienyl, indenocarbazolyl, indolocarbazolyl, phenanthryl or triphenylenyl, which may in each case be substituted by one or more radicals R¹.

5. Process according to one or more of Claims 1 to 4, **characterised in that** the aryl compound is substituted by a leaving group and the leaving group or the group X in the compound of the formula (2) is selected from the group consisting of optionally substituted alkylsulfonate, optionally substituted arylsulfonate, halide and diazonium.

6. Process according to Claim 5, **characterised in that** the aryl compound is substituted by a leaving group and the leaving group or the group X in the compound of the formula (2) is selected from the group consisting of triflate, phenylsulfonate and tosylate.

7. Process according to one or more of Claims 1 to 6, **characterised in that** the aromatic or heteroaromatic ring system in the aryl compound or the group Ar in the compound of the formula (2) is selected from the group consisting of phenyl, biphenyl, terphenyl, quaterphenyl, fluorenyl, spirobifluorenyl, naphthyl, indolyl, benzofuranyl, benzothienyl, carbazolyl, dibenzofuranyl, dibenzothienyl, indenocarbazolyl, indolocarbazolyl, phenanthrenyl or triphenylenyl, which may in each case be substituted by one or more radicals R¹.

8. Process according to one or more of Claims 1 to 7, **characterised in that** lithium tert-butoxide is employed in an amount of 0.5 to 10 equivalents, based on the molar amount of aryl compound employed.

9. Process according to one or more of Claims 1 to 8, **characterised in that** the catalyst employed is PdCl₂, Pd(OAc)₂, (CH₃CN)₂PdCl₂, bis-(dibenzylideneacetone)dipalladium or tris(dibenzylideneacetone)-dipalladium together with at least one ligand.

10. Process according to one or more of Claims 1 to 9, **characterised in that** the ligand employed for the catalyst is a phosphine, phosphite, amine, aminophosphine or N-heterocyclic carbene.

11. Process according to Claim 10, **characterised in that** the phosphine is selected from the group consisting of dicyclohexylphosphino-2',6'-dimethoxybiphenyl, dicyclohexylphosphino-2',6'-diisopropoxybiphenyl, di-tert-butyl(2',4',6'-triisopropyl-3,6-dimethoxybiphenyl-2-yl)phosphine, dicyclohexyl(2',4',6'-triisopropyl-3,6-dimethoxybiphenyl-2-yl)phosphine, trimethylphosphine, triethylphosphine, tripropylphosphine, triisopropylphosphine, tributylphosphine, tri-tert-butyl-phosphine, tricyclohexylphosphine, triphenylphosphine, di-tert-butylchlorophosphine, triphenylphosphine, tri(o-tolyl)phosphine, triisopropylphosphine, tricyclohexylphosphine, 2,2'-bis(diphenylphosphino)-1,1'-binaphthyl (BINAP), 1,2-bis(dimethylphosphino)ethane, 1,2-bis(diethylphosphino)ethane, 1,2-bis(dipropylphosphino)ethane, 1,2-bis(diisopropylphosphino)ethane, 1,2-bis(dibutylphosphino)ethane, 1,2-bis(dicyclohexylphosphino)-ethane, 1,3-bis(dicyclohexylphosphino)propane, 1,3-bis(diisopropyl-phosphino)propane, 1,4-bis(diisopropylphosphino)butane, 2,4-bis-(dicyclohexylphosphino)pentane, 1,1 '-bis(diphenylphosphino)-ferrocene, SPhos, PCy₃, Cy-JohnPhos, CataCxium Pcy, APhos, XantPhos, dppf, XPhos and BrettPhos.

12. Process according to one or more of Claims 9 to 11, **characterised in that** for monophosphines the palladium compound and the phosphine ligand are employed in a Pd : phosphine ratio of 1 : 1 to 1 : 4 and **in that** for biphosphines the palladium compound and the phosphine ligand are employed in a Pd : phosphine ratio of 1 : 0.5 to 1 : 2.

13. Process according to one or more of Claims 1 to 12, **characterised in that** the process is carried out in one or more aprotic organic solvents, preferably selected from benzene, toluene, 1,2-xylene, 1,3-xylene, 1,4-xylene, mesitylene, tetrahydrofuran (THF), 1,4-dioxane, dimethoxyethane (DME) and bis(2-methoxyethyl) ether (diglyme).

14. Process according to one or more of Claims 1 to 13, **characterised in that** the process is carried out under an inert-gas atmosphere.

15. Use of LiOtBu as base in a palladium-catalysed coupling reaction between an arylamino compound and an aryl compound.

## Revendications

1. Procédé de préparation d'un composé arylamino secondaire ou tertiaire par une réaction de couplage catalysée au palladium entre un composé arylamino primaire ou secondaire et un composé aryle, **caractérisé en ce que** le procédé est effectué en présence de tertio-butylate de lithium (LiOtBu) comme base.

2. Procédé selon la revendication 1, **caractérisé en ce que** le composé arylamino employé est un composé de formule (1), 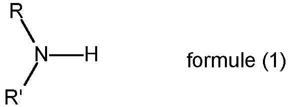 dans laquelle ce qui suit s'applique aux symboles utilisés :
R est, de manière identique ou différente à chaque occurrence, un noyau aromatique ou hétéroaromatique ayant de 5 à 60 atomes de cycle aromatique, qui peut dans chaque cas être substitué par un ou plusieurs radicaux R¹ ;
R' est, de manière identique ou différente, R ou représente H ou D ; R et R' peuvent être liés l'un à l'autre directement ou via un groupement R¹ et former ainsi un noyau conjointement avec l'atome d'azote auquel ils sont liés ;
R¹ est choisi, de manière identique ou différente à chaque occurrence, parmi H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R² et où un ou plusieurs groupements CH₂ dans les groupements susmentionnés peuvent être remplacés par -R²C=CR²-, -C=C-, Si(R²)₂, C=O, C=S, C=NR², -C(=O)O-, -C(=O)NR²-, NR², P(=O)(R²), -O-, -S-, SO ou SO₂ et où un ou plusieurs atomes de H dans les groupements susmentionnés peuvent être remplacés par D, F, Cl, Br, I, CN ou NO₂, ou un noyau aromatique ou hétéroaromatique ayant de 5 à 30 atomes de cycle aromatique, qui peut dans chaque cas être substitué par un ou plusieurs radicaux R², ou un groupement aryl- ou hétéroaryloxy ayant de 5 à 30 atomes de cycle aromatique, qui peut être substitué par un ou plusieurs radicaux R² ; deux, ou plus, radicaux R¹ peuvent être liés l'un à l'autre et former ainsi un cycle ;
R² est choisi, de manière identique ou différente à chaque occurrence, parmi H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R³ et où un ou plusieurs groupements CH₂ dans les groupements susmentionnés peuvent être remplacés par -R³C=CR³-, -C=C-, Si(R³)₂, C=O, C=S, C=NR³, -C(=O)O-, -C(=O)NR³-, NR³, P(=O)(R³), -O-, -S-, SO ou SO₂ et où un ou plusieurs atomes de H dans les groupements susmentionnés peuvent être remplacés par D, F, Cl, Br, I, CN ou NO₂, ou un noyau aromatique ou hétéroaromatique ayant de 5 à 30 atomes de cycle aromatique, qui peut dans chaque cas être substitué par un ou plusieurs radicaux R³, ou un groupement aryl- ou hétéroaryloxy ayant de 5 à 30 atomes de cycle aromatique, qui peut être substitué par un ou plusieurs radicaux R³ ; deux, ou plus, radicaux R² peuvent être liés l'un à l'autre et former ainsi un cycle ;
R³ est à chaque occurrence, de manière identique ou différente, H, D, F ou un radical organique aliphatique, aromatique ou hétéroaromatique ayant de 1 à 20 atomes de C, dans lequel, de plus, un ou plusieurs atomes de H peuvent être remplacés par D ou F ; deux, ou plus, substituants R³ peuvent être liés l'un à l'autre et former ainsi un cycle.

3. Procédé selon la revendication 1 ou 2, **caractérisé en ce que** le composé aryle employé est un composé de formule (2), 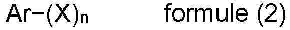 dans laquelle R¹, R² et R³ revêtent les significations données selon la revendication 2 et ce qui suit s'applique aux autres symboles et indices utilisés :
Ar est un noyau aromatique ou hétéroaromatique ayant de 5 à 60 atomes de cycle aromatique, qui peut être substitué par un ou plusieurs radicaux R¹ ;
X est à chaque occurrence, de manière identique ou différente, un groupement partant ;
n est un nombre entier allant de 1 à 10.

4. Procédé selon la revendication 2 ou 3, **caractérisé en ce que** R et R', si R' ne représente pas H ou D, sont choisis, de manière identique ou différente à chaque occurrence, dans le groupe constitué par phényle, biphényle, terphényle, quaterphényle, fluorényle, spirobifluorényle, naphtyle, indolyle, benzofuranyle, benzothiényle, carbazolyle, dibenzofuranyle, dibenzothiényle, indénocarbazolyle, indolocarbazolyle, phénanthryle ou triphénylényle, qui peuvent dans chaque cas être substitués par un ou plusieurs radicaux R¹.

5. Procédé selon l'une ou plusieurs parmi les revendications 1 à 4, **caractérisé en ce que** le composé aryle est substitué par un groupement partant et le groupement partant ou le groupement X dans le composé de formule (2) est choisi dans le groupe constitué par alkylsulfonate éventuellement substitué, arylsulfonate éventuellement substitué, halogénure et diazonium.

6. Procédé selon la revendication 5, **caractérisé en ce que** le composé aryle est substitué par un groupement partant et le groupement partant ou le groupement X dans le composé de formule (2) est choisi dans le groupe constitué par triflate, phénylsulfonate et tosylate.

7. Procédé selon l'une ou plusieurs parmi les revendications 1 à 6, **caractérisé en ce que** le noyau aromatique ou hétéroaromatique dans le composé aryle ou le groupement Ar dans le composé de formule (2) est choisi dans le groupe constitué par phényle, biphényle, terphényle, quaterphényle, fluorényle, spirobifluorényle, naphtyle, indolyle, benzofuranyle, benzothiényle, carbazolyle, dibenzofuranyle, dibenzothiényle, indénocarbazolyle, indolocarbazolyle, phénanthrényle ou triphénylényle, qui peut dans chaque cas être substitué par un ou plusieurs radicaux R¹.

8. Procédé selon l'une ou plusieurs parmi les revendications 1 à 7, **caractérisé en ce que** le tertio-butylate de lithium est employé selon une quantité allant de 0,5 à 10 équivalents, sur la base de la quantité molaire du composé aryle employé.

9. Procédé selon l'une ou plusieurs parmi les revendications 1 à 8, **caractérisé en ce que** le catalyseur employé est PdCl₃, Pd(OAc)₂, (CH₃CN)₂PdCl₂, le bis(dibenzylidèneacétone)dipalladium ou le tris-(dibenzylidèneacétone)dipalladium conjointement avec au moins un ligand.

10. Procédé selon l'une ou plusieurs parmi les revendications 1 à 9, **caractérisé en ce que** le ligand employé pour le catalyseur est une phosphine, un phosphite, une amine, une aminophosphine ou un carbène N-hétérocyclique.

11. Procédé selon la revendication 10, **caractérisé en ce que** la phosphine est choisie dans le groupe constitué par le dicyclohexylphosphino-2',6'-diméthoxybiphényle, le dicyclohexylphosphino-2',6'-diisopropoxy-biphényle, la di-tertio-butyl(2',4',6'-triisopropyl-3,6-diméthoxybiphényl-2-yl)phosphine, la dicyclohexyl(2',4',6'-triisopropyl-3,6-diméthoxybiphé-nyl-2-yl)phosphine, la triméthylphosphine, la triéthylphosphine, la tripropylphosphine, la triisopropylphosphine, la tributylphosphine, la tritertio-butyl-phosphine, la tricyclohexylphosphine, la triphénylphosphine, la di-tertio-butylchlorophosphine, la triphénylphosphine, la tri(o-tolyl)phosphine, la triisopropylphosphine, la tricyclohexylphosphine, le 2,2'-bis(diphénylphosphino)-1,1'-binaphtyle (BINAP), le 1,2-bis(dimé-thylphosphino)éthane, le 1,2-bis(diéthylphosphino)éthane, le 1,2-bis(dipropylphosphino)éthane, le 1,2-bis(diisopropylphosphino)éthane, le 1,2-bis(dibutylphosphino)éthane, le 1,2-bis(dicyclohexylphosphino)éthane, le 1,3-bis(dicyclohexylphosphino)propane, le 1,3-bis(diisopropylphosphino)propane, le 1,4-bis(diisopropylphosphino)butane, le 2,4-bis(dicyclohexylphosphino)pentane, le 1,1'-bis(diphénylphosphino)ferrocène, SPhos, PCy₃, Cy-JohnPhos, CataCxium Pcy, APhos, XantPhos, dppf, XPhos et BrettPhos.

12. Procédé selon l'une ou plusieurs parmi les revendications 9 à 11, **caractérisé en ce que** pour les monophosphines, le composé de palladium et le ligand phosphine sont employés selon un rapport Pd:phosphine allant de 1:1 à 1:4 et **en ce que** pour les biphosphines, le composé de palladium et le ligand phosphine sont employés selon un rapport Pd:phosphine allant de 1:0,5 à 1:2.

13. Procédé selon l'une ou plusieurs parmi les revendications 1 à 12, **caractérisé en ce que** le procédé est effectué dans un ou plusieurs solvants organiques aprotiques, choisis de préférence parmi le benzène, le toluène, le 1,2-xylène, le 1,3-xylène, le 1,4-xylène, le mésitylène, le tétrahydrofurane (THF), le 1,4-dioxane, le diméthoxyéthane (DME) et le bis(2-méthoxyéthyl) éther (diglyme).

14. Procédé selon l'une ou plusieurs parmi les revendications 1 à 13, **caractérisé en ce que** le procédé est effectué sous une atmosphère de gaz inerte.

15. Utilisation de LiOtBu comme base dans une réaction de couplage catalysée au palladium entre un composé arylamino et un composé aryle.
